# EUROPEAN PATENT APPLICATION

(11) **EP 4 814 012 A1**
(43) Date of publication of application: **30.09.2026**
(21) Application number: 24893549.6
(22) Date of filing: 21.11.2024
(51) Int. Cl.: C07K 16/24, C12N 15/13, A61K 39/395

(54) **ANTI-IL-4R-ALPHA ANTIBODY OR ANTIGEN-BINDING FRAGMENT THEREOF, AND BISPECIFIC ANTIBODY AND USE THEREOF**

(30) Priority: 22.11.2023 CN 202311565388
(71) Applicant: The United Bio-Technology (Hengqin) Co., Ltd., Zhuhai, Guangdong 519031 (CN)
(72) Inventor: LI, Jing, Zhuhai, Guangdong 519031 (CN); CHEN, Zongmeng, Zhuhai, Guangdong 519031 (CN); CAO, Chunlai, Zhuhai, Guangdong 519031 (CN); HE, Hua, Zhuhai, Guangdong 519031 (CN); WU, Qiang, Zhuhai, Guangdong 519031 (CN); LI, Wendan, Zhuhai, Guangdong 519031 (CN); SU, Lijuan, Zhuhai, Guangdong 519031 (CN); CHEN, Kangyue, Zhuhai, Guangdong 519031 (CN); ZHANG, Xueying, Zhuhai, Guangdong 519031 (CN); ZHANG, Minghao, Zhuhai, Guangdong 519031 (CN); ZHOU, Cui, Zhuhai, Guangdong 519031 (CN); WANG, Jiayu, Zhuhai, Guangdong 519031 (CN)
(74) Representative: Michalski Hüttermann & Partner mbB
(86) International application number: PCT/CN2024/133628
(87) International publication number: WO 2025/108394

(57) **Abstract**

The present invention relates to the field of biomedicine. Provided are an anti-human IL-4Rα antibody or an antigen-binding fragment thereof and the use thereof, The anti-human IL-4Rα anti-body comprises heavy chain complementarity determination regions HCDR1, HCDR2 and HCDR3, and light chain complementarity determination regions LCDR1, LCDR2 and LCDR3, has high affinity with human IL-4Rα, can be used for preparing an antibody drug, can be used for treating type-II inflammatory diseases, and has relatively good clinical efficacy and relatively low toxic and side effects. Further provided are a bispecific antibody against both IL-4Rα and TSLP, and the use of the antibody in the preparation of a drug for treating severe asthma, moderate to severe atopic dermatitis and chronic obstructive pulmonary diseases.

## Description

### TECHNICAL FIELD

The present invention relates to the field of biomedicine, and in particular, to an antibody binding to human IL-4Rα, an antigen-binding fragment thereof, a bispecific antibody thereof, and uses thereof.

### BACKGROUND ART

Interleukin-4 (IL-4) is mainly secreted by activated T cells, monocytes, basophilic granulocyte, mast cells and eosinophils. Its biological functions include stimulating the proliferation of B cells and T cells, as well as T cell differentiation. Interleukin-13 (IL-13) is expressed by a variety of cells. It can inhibit the release of inflammatory cytokines from monocytes, induce the proliferation and differentiation of B cells, promote antibody secretion by B cells, enhance IgE expression, and is associated with allergic reactions in the body. Both IL-4 and IL-13 exert regulatory effects by binding to the common functional receptor IL-4Rα. IL-4 functions through type I receptors complex (IL-4Rα/γc) and type II receptors complex (IL-4Rα/IL-13Rα1). γc is expressed only on the hematopoietic cells, while IL-13Rα1 is expressed on both hematopoietic cells and non-hematopoietic cells (such as airway epithelial cells). IL-13 functions only through type II receptors complex. Upon binding of IL-4 or IL-13 to the receptor, receptor dimerization further induces phosphorylation and activation of JAKs proteins, including JAK1, JAK3, and JAK2, which correspond to IL-4Rα, γc, and IL-13Rα1, respectively. This is followed by promoting phosphorylation of specific tyrosine residues in the intracellular domain of IL-4Rα, which in turn phosphorylates the transcription factor STAT6, thereby promoting gene transcription and protein expression.

Type 2 inflammatory diseases mainly include atopic dermatitis, asthma, allergic rhinitis, chronic nasosinusitis with nasal polyps, chronic obstructive pulmonary disease, which are usually caused by chronic imbalance of innate and adaptive immune systems. Environmental triggers, genetic factors and barrier disruption continuously activate adaptive immune cells (Th2) to produce cytokines such as IL-4, IL-5 and IL-13. These cytokines drive B-cell class switching and IgE production, promote the differentiation and migration of eosinophils, cause degranulation of basophilic granulocyte and mast cells, and exacerbate the development and progression of inflammation. IL-4Rα is a key factor in the type 2 immune response, which can simultaneously mediate the functions of IL-4 and IL-13 cytokines. Therefore, blocking IL-4Rα can simultaneously inhibit IL-4 and IL-13 immune responses.

Currently, monoclonal antibody drugs targeting human IL-4Rα have demonstrated excellent therapeutic effects in clinical studies of multiple indications, fully validating the druggability of this target. Developing antibody drugs with high affinity for human IL-4Rα for the treatment of type 2 inflammatory diseases-thereby achieving better clinical efficacy, lower toxicity and side effects, and providing an additional therapeutic option for patients-is of great significance.

Dupilumab is the first marketed monoclonal antibody drug targeting IL-4Rα. It exhibits significant clinical efficacy and has been approved for the treatment of multiple indications. However, it still has limitations in clinical application, including limited efficacy in patients with atopic dermatitis, low response rate, and adverse reaction in some patients caused by increased eosinophils during treatment. From the perspective of clinical evaluation indicators for Dupilumab therapy, the reduction of eosinophil counts following Dupilumab treatment is limited, an observation that aligns with the molecular mechanism of IL-4Rα.

Thymic stromal lymphopoietin (TSLP), belonging to the interleukin-7 family, is produced by epithelial cells, smooth muscle cells, stromal cells, macrophages, mononuclear macrophages. Numerous studies have shown that TSLP is located at the apex of multiple inflammatory cascades and serves as a key driver factor of Th2-type inflammatory responses. Upon stimulation or disruption of epithelial cells, dendritic cells present antigens to naive CD4+T cells and secrete epithelial cytokines, TSLP, and IL-33. These co-stimulatory cytokines promote the differentiation of allergen-specific Th2 cells, leading to the production of cytokines such as IL-4, IL-5, and IL-13. Thus, TSLP is generally considered to be one of the key triggers of inflammatory cascade. Inhibition of TSLP allows for early intervention in the inflammation process, preventing immune cells from releasing pro-inflammatory cytokines, thereby being more effective than inhibiting IL-4, IL-5 and IL-13 alone. In addition, in vitro and in vivo pharmacological evidence suggests that TSLP is also involved in non-Th2 inflammatory and may contribute to Th1/Th17-related autoimmune diseases, such as rheumatoid arthritis and multiple sclerosis. Tezepelumab is currently the only marketed monoclonal antibody drug targeting TSLP, approved for the treatment of moderate-to-severe asthma in adult patients with excellent therapeutic efficacy. However, it also has limitations in clinical application, as it failed to meet the primary endpoints in clinical studies for atopic dermatitis. Comprehensive analysis of serological changes in patients treated with Tezepelumab showed that the eosinophils counts decreased by approximately 50%, levels of both IL-5 and IL-13 decreased by more than 50%, while IgE levels were only with a reduction of about 20%, and its modulation of IL-4 was weak.

Bispecific antibodies can simultaneously target two different antigens, broadening the molecular action spectrum while maintaining the high specificity of antibodies, thereby improving clinical outcomes. A variety of bispecific antibody formats have been developed currently, among which the IgG-scFv format maintains the intact IgG structure of natural monoclonal antibodies, and offers superior advantages in molecular design, expression and purification, making it an ideal format for bifunctional antibodies.

Comprehensive analysis of clinical effects of inhibiting the two targets shows that Dupilumab has limited effect on reduction of eosinophils counts, while Tezepelumab exhibits limited effect on the reduction of IL-4 and IgE level. The regulation of the Th2 signaling pathway involves multiple cytokines. Simultaneous inhibition of both targets can theoretically significantly improve the clinical response rate and expand the scope of indications. Therefore, there remains a clinical need to develop novel bispecific antibodies against IL-4Rα and TSLP. By taking advantage of the complementary inhibitory effects of the two targets, they can achieve better therapeutic efficacy and higher safety.

### SUMMARY

In view of the above status, the present invention provides an anti-IL-4Rα antibody or an antigen-binding fragment thereof, as well as a bispecific antibody thereof. The antibody of the present invention are high affinity, favorable activity and low hydrophobicity, and can be used for the treatment of allergic diseases such as atopic dermatitis and asthma.

The anti-IL-4Rα antibody or antigen-binding fragment thereof according to the present invention comprises heavy chain complementarity-determining regions and light chain complementarity-determining regions,

The heavy chain complementarity-determining regions include HCDR1, HCDR2, and HCDR3, wherein the HCDR1 is the amino acid sequence shown in SEQ ID NO: 1 or SEQ ID NO: 12; the HCDR2 is the amino acid sequence shown in SEQ ID NO: 2, SEQ ID NO: 7, SEQ ID NO: 10, or SEQ ID NO: 13; the HCDR3 is the amino acid sequence shown in SEQ ID NO: 3, SEQ ID NO: 9, or SEQ ID NO: 14; and/or

The light chain complementarity-determining regions include LCDR1, LCDR2, and LCDR3, wherein the LCDR1 is the amino acid sequence shown in SEQ ID NO: 4 or SEQ ID NO: 15; the LCDR2 is the amino acid sequence shown in SEQ ID NO: 5 or SEQ ID NO: 16; the LCDR3 is the amino acid sequence shown in SEQ ID NO: 6, SEQ ID NO: 8, SEQ ID NO: 11, or SEQ ID NO: 17.

In the present invention, as one of the embodiments, the anti-IL-4Rα antibody or antigen-binding fragment thereof comprises:
(1) a heavy chain complementarity-determining region comprising HCDR1 of the amino acid sequence shown in SEQ ID NO: 1, HCDR2 of the amino acid sequence shown in SEQ ID NO: 2, and HCDR3 of the amino acid sequence shown in SEQ ID NO: 3; and
   a light chain complementarity-determining region comprising LCDR1 of the amino acid sequence shown in SEQ ID NO: 4, LCDR2 of the amino acid sequence shown in SEQ ID NO: 5, and LCDR3 of the amino acid sequence shown in SEQ ID NO: 6.

In the present invention, as one of the embodiments, the anti-IL-4Rα antibody or antigen-binding fragment thereof comprises:
(2) a heavy chain complementarity-determining region comprising HCDR1 of the amino acid sequence shown in SEQ ID NO: 1, HCDR2 of the amino acid sequence shown in SEQ ID NO: 7, and HCDR3 of the amino acid sequence shown in SEQ ID NO: 3; and
a light chain complementarity-determining region comprising LCDR1 of the amino acid sequence shown in SEQ ID NO: 4, LCDR2 of the amino acid sequence shown in SEQ ID NO: 5, and LCDR3 of the amino acid sequence shown in SEQ ID NO: 8.

In the present invention, as one of the embodiments, the anti-IL-4Rα antibody or antigen-binding fragment thereof comprises:
(3) a heavy chain complementarity-determining region comprising the amino acid sequences of HCDR1 shown in SEQ ID NO: 1, HCDR2 shown in SEQ ID NO: 7, and HCDR3 shown in SEQ ID NO: 9; and,
a light chain complementarity-determining region comprising the amino acid sequences of LCDR1 shown in SEQ ID NO: 4, LCDR2 shown in SEQ ID NO: 5, and LCDR3 shown in SEQ ID NO: 8.

In the present invention, as one of the embodiments, the anti-IL-4Rα antibody or antigen-binding fragment thereof comprises:
(4) a heavy chain complementarity-determining region comprising the amino acid sequences of HCDR1 shown in SEQ ID NO: 1, HCDR2 shown in SEQ ID NO: 10, and HCDR3 shown in SEQ ID NO: 3; and
a light chain complementarity-determining region comprising the amino acid sequences of LCDR1 shown in SEQ ID NO: 4, LCDR2 shown in SEQ ID NO: 5, and LCDR3 shown in SEQ ID NO: 11.

In the present invention, as one of the embodiments, the anti-IL-4Rα antibody or antigen-binding fragment thereof comprises:
(5) a heavy chain complementarity-determining region comprising the amino acid sequences of HCDR1 shown in SEQ ID NO: 12, HCDR2 shown in SEQ ID NO: 13, and HCDR3 shown in SEQ ID NO: 14; and
a light chain complementarity-determining region comprising the amino acid sequences of LCDR1 shown in SEQ ID NO: 15, LCDR2 shown in SEQ ID NO: 16, and LCDR3 shown in SEQ ID NO: 17.

The above-mentioned anti-IL-4Rα antibody or antigen-binding fragment thereof further comprises a fragment selected from:
A framework region (FR) sequence derived from the human germline heavy chain IGHV1-46*01 + IGHJ1*01, or a back-mutated sequence having at least 90% identity thereof; A framework region sequence derived from the human germine light chain IGKV1-39*01+IGKJ2*01, or a back-mutated sequence having at least 90% identity thereof.

The above-mentioned anti-IL-4Rα antibody or antigen-binding fragment thereof provided by the present invention is a murine antibody, a chimeric antibody, a human antibody, a humanized antibody, a bispecific antibody, or a fragment thereof.

The above-mentioned anti-IL-4Rα antibody or antigen-binding fragment thereof provided by the present invention comprises:
(1) a variable region of heavy chain comprising the amino acid sequence shown in SEQ ID NO: 28 and a variable region of light chain comprising the amino acid sequence shown in SEQ ID NO: 29;
(2) a variable region of heavy chain comprising the amino acid sequence shown in SEQ ID NO: 30 and a variable region of light chain comprising the amino acid sequence shown in SEQ ID NO: 31;
(3) a variable region of heavy chain comprising the amino acid sequence shown in SEQ ID NO: 32 and a variable region of light chain comprising the amino acid sequence shown in SEQ ID NO: 33;
(4) a variable region of heavy chain comprising the amino acid sequence shown in SEQ ID NO: 18 and a variable region of light chain comprising the amino acid sequence shown in SEQ ID NO: 19;
(5) a variable region of heavy chain comprising the amino acid sequence shown in SEQ ID NO: 20 and a variable region of light chain comprising the amino acid sequence shown in SEQ ID NO: 21;
The above-mentioned anti-IL-4Rα antibody or antigen-binding fragment thereof provided by the present invention comprises an amino acid sequence having 90% or more identity.

The present invention also provides a fusion protein, comprising the above-mentioned anti-IL-4Rα antibody or antigen-binding fragment thereof.

The present invention also provides a polynucleotide encoding the above-mentioned anti-IL-4Rα antibody or antigen-binding fragment thereof.

The present invention also provides an expression vector comprising the above-mentioned polynucleotide.

The present invention also provides a host cell comprising the above-mentioned vector, wherein the host cell is a prokaryotic or eukaryotic cell.

The present invention also provides a pharmaceutical composition comprising the anti-IL-4Rα antibody or antigen-binding fragment thereof or the above-mentioned fusion protein and a pharmaceutically acceptable excipient.

The present invention also provides use of the anti-IL-4Rα antibody or antigen-binding fragment thereof, the above-mentioned fusion protein, or the pharmaceutical composition thereof in the preparation of a medicament for treating allergic diseases.

In the present invention, as one of the embodiments, the above-mentioned allergic disease is selected from atopic dermatitis, asthma, allergic rhinitis, certain cases of chronic nasosinusitis with nasal polyps, or chronic obstructive pneumonia.

The anti-IL-4Rα antibody or antigen-binding fragment thereof provided by the present invention, which differs from the prior art, is an antibody drug with high affinity for human IL-4Rα, used for the treatment of type 2 inflammatory diseases. It offers better clinical efficacy, lower toxic side effects, and provides patients with an additional drug option, which is of great significance.

As detected by surface plasmon resonance, the m8, m21, and m27 screened in the present invention have affinities comparable to that of the Dupilumab antibody, wherein the KD value of the m8 molecule is 4.37E-11 M, with the KD value being below 50 pM.

The antibody molecules m21 and m27 screened in the present invention are superior to the Dupilumab antibody in inhibiting hIL-13-induced proliferation of TF-1 cells, while the m8 antibody molecule exhibits a blocking activity comparable to that of Dupilumab.

As determined by hydrophobic interaction chromatography, the retention times of the m8, m21, and m27 molecules screened in the present invention are significantly lower than that of the positive antibody BMK1, indicating that their hydrophobicity is significantly lower than that of the Dupilumab antibody. Antibodies with low hydrophobicity are less prone to form aggregation during process development and are less likely to form unstable complex by binding to host cell proteins or other impurities. This offers a significant advantage for subsequent process development.

The present invention also provides a bispecific antibody which is capable of binding human IL-4Rα and TSLP simultaneously, wherein the bispecific antibody adopts an IgG-scFv format and the bispecific antibody comprises an anti-IL-4Rα antibody or an antigen-binding fragment thereof as defined in any one of the preceding items.

The bispecific antibody of the present invention comprises an immunoglobulin IgG and a single-chain antibody scFv, wherein the bispecific antibody is selected from one of the following groups:
(1) The immunoglobulin IgG comprises an anti-TSLP antibody or an antigen-binding fragment thereof, and the single-chain antibody scFv comprises an anti-IL-4Rα antibody or an antigen-binding fragment thereof as defined in any one of the preceding items; or
(2) The immunoglobulin IgG comprises an anti-IL-4Rα antibody or an antigen-binding fragment thereof as defined in any one of the preceding items, the single-chain antibody scFv comprises an anti-TSLP antibody or an antigen-binding fragment thereof.

As one of the embodiments, the above-mentioned bispecific antibody of the present invention, wherein the anti-TSLP antibody or antigen-binding fragment thereof comprises a variable region of heavy chain and a variable region of light chain, wherein
The variable region of heavy chain comprises HCDR1, HCDR2, and HCDR3, wherein HCDR1 is the amino acid sequence shown in SEQ ID NO: 48; HCDR2 is the amino acid sequence shown in SEQ ID NO: 49; and HCDR3 is the amino acid sequence shown in SEQ ID NO: 50;
The variable region of light chain comprises LCDR1, LCDR2, and LCDR3, wherein LCDR1 is the amino acid sequence shown in SEQ ID NO: 51; the amino acid sequence of LCDR2 is DDS; and LCDR3 is the amino acid sequence shown in SEQ ID NO: 52.

As one of the embodiments, the bispecific antibody of the present invention, wherein, the anti-IL-4Rα antibody or antigen-binding fragment thereof comprises a variable region of heavy chain and a variable region of light chain, wherein,
The variable region of heavy chain comprises HCDR1, HCDR2, and HCDR3, wherein the HCDR1 is the amino acid sequence shown in SEQ ID NO:1; the HCDR2 is the amino acid sequence shown in SEQ ID NO:2; and HCDR3 is the amino acid sequence shown in SEQ ID NO:3;
The variable region of light chain comprises LCDR1, LCDR2, and LCDR3, wherein the LCDR1 is the amino acid sequence shown in SEQ ID NO:4; the LCDR2 is the amino acid sequence shown in SEQ ID NO:5; and the LCDR3 is the amino acid sequence shown in SEQ ID NO:6.

In the present invention, as one of the embodiments, the anti-TSLP antibody or antigen-binding fragment thereof comprises a variable region of heavy chain and a variable region of light chain, wherein,
The variable region of heavy chain comprises the amino acid sequence shown in SEQ ID NO: 42, the variable region of light chain comprises the amino acid sequence shown in SEQ ID NO: 43, or,
The variable region of heavy chain comprises the amino acid sequence shown in SEQ ID NO: 34, and the variable region of light chain comprises the amino acid sequence shown in SEQ ID NO: 36.

In the present invention, as one of the embodiments, the anti-IL-4Rα antibody or antigen-binding fragment thereof comprises a variable region of heavy chain and a variable region of light chain, wherein,
The variable region of heavy chain has the amino acid sequence shown in SEQ ID NO:44, the variable region of light chain has the amino acid sequence shown in SEQ ID NO:45, or
The variable region of heavy chain has the amino acid sequence shown in SEQ ID NO:28 the variable region of light chain has the amino acid sequence shown in SEQ ID NO:29.

In the present invention, as one of the embodiments, the immunoglobulin IgG and the single-chain antibody scFv are connected via a linker, wherein the linker is (GGGGS)n, where n is a positive integer from 1 to 5, preferably the amino acid sequence shown in SEQ ID NO: 46. For exemplary illustration purpose, n may be 1, 2, 3, 4, or 5.

In the present invention, as one of the embodiments, the single-chain antibody scFv further comprises a linker connecting the heavy-chain variable region and the light-chain variable region of the scFv. The linker is (GGGGS)n, where n is a positive integer from 1 to 5, preferably the amino acid sequence shown in SEQ ID NO: 47. For exemplary illustration purpose, n may be 1, 2, 3, 4, or 5.

As one of the embodiments, the bispecific antibody of the present invention is selected from any one of the following groups:
(1) The immunoglobulin IgG comprises a variable region of heavy chain with the amino acid sequence as shown in SEQ ID NO: 34, a constant region of heavy chain as shown in SEQ ID NO: 35, a variable region of light chain as shown in SEQ ID NO: 36, and a constant region of light chain with the amino acid sequence as shown in SEQ ID NO: 37, the single-chain antibody scFv portion comprises a variable region of heavy chain with an amino acid sequence as shown in SEQ ID NO:44 and a variable region of light chain with an amino acid sequence as shown in SEQ ID NO:45; or
(2) The immunoglobulin IgG comprises a variable region of heavy chain with the amino acid sequence as shown in SEQ ID NO: 28, a constant region of heavy chain as shown in SEQ ID NO: 40, a variable region of light chain as shown in SEQ ID NO: 29, and a constant region of light chain with the amino acid sequence as shown in SEQ ID NO: 41, the single-chain antibody scFv portion comprises a variable region of heavy chain with an amino acid sequence as shown in SEQ ID NO:42 and a variable region of light chain with an amino acid sequence as shown in SEQ ID NO:43.

As one of the embodiments, the bispecific antibody of the present invention is selected from any one of the following groups:
(1) The bispecific antibody comprises a heavy chain with an amino acid sequence as shown in SEQ ID NO: 57 and a light chain with an amino acid sequence as shown in SEQ ID NO: 58; or
(2) The bispecific antibody comprises a heavy chain with an amino acid sequence as shown in SEQ ID NO: 59 and a light chain with an amino acid sequence as shown in SEQ ID NO: 60.

The present invention also provides a pharmaceutical composition, comprising the above-mentioned bispecific antibody or an antigen-binding fragment thereof and pharmaceutically acceptable excipients.

The present invention also provides the use of the bispecific antibody according to any one of the above items or the above-mentioned pharmaceutical composition in the preparation of a medicament for the treatment of moderate to severe asthma, moderate to severe atopic dermatitis, allergic rhinitis, certain cases of chronic nasosinusitis with nasal polyps, and chronic obstructive pulmonary disease.

The bispecific antibodies MTM and MMT provided by the present invention exhibit comparable binding affinity to hIL-4Rα as Dupilumab, and comparable binding affinity to hTSLP as Tezepelumab. The bispecific antibodies MTM and MMT provided by the present invention can effectively block the activity of hIL-13 and hIL-4, and exhibit comparable activity to Dupilumab. A GFP reporter gene assay targeting IL-4Rα was used to evaluate the ability of the bispecific antibodies to block hIL-13 and hIL-4. The results demonstrated that MTM and MMT can effectively block the activity of hIL-13 and hIL-4.

The bispecific antibodies MTM and MMT provided by the present invention can effectively block the activity of TSLP and exhibit comparable activity to Tezepelumab. A luciferase reporter gene assay for TSLP activity evaluation was employed to assess the ability of the bispecific antibodies to block hTSLP. The results demonstrated that MTM and MMT can effectively block the activity of hTSLP.

The bispecific antibody MTM provided by the present invention exhibits favorable stability. In a buffer of 20 mM sodium acetate + 100 mM L-proline, pH 5.0, the MTM molecule can be concentrated to a concentration of over 100 mg/mL. Stability studies were conducted at 4°C, 25°C, and 40°C, using the monomer determined by SEC-HPLC as the evaluation indicator. The results demonstrated that the MTM molecule has favorable stability, which is beneficial for subsequent process development.

### BRIEF DESCRIPTION OF THE DRAWINGS

Figure 1A: FACS determination of the binding activity of the first batch of murine antibody molecules to CHO-K1-hIL-4Rα;
Figure 1B: FACS determination of the binding activity of the second batch of murine antibody molecules to CHO-K1-hIL-4Rα;
Figure 2A: Inhibitory effect of the first group of candidate murine antibody molecules on hIL-13 determined by reporter gene assay;
Figure 2B: Inhibitory effect of the second group of candidate murine antibody molecules on hIL-13 determined by reporter gene assay;
Figure 3: Inhibitory effect of affinity-matured monoclonal antibody molecules on hIL-4 activity as determined by reporter gene assay;
Figure 4: Pharmacodynamic results of monoclonal antibodies m8 and m21 in a B-hIL4/IL4Rα humanized mouse asthma model;
Figure 5: Purity of monoclonal antibodies m8 and m21 determined by SEC-HPLC;
Figure 6: Hydrophobicity of monoclonal antibodies m8 and m21 determined by HIC-HPLC;
Figure 7: Purity of bispecific antibody molecules determined by SDS-PAGE;
Figure 8A: Simultaneous binding ability of MMT molecule to hIL-4Rα and hTSLP antigens determined by Biacore 8k;
Figure 8B: Simultaneous binding ability of MTM molecule to hIL-4Rα and hTSLP antigens determined by Biacore 8k;
Figure 9: Quantification of CCL17 secreted by human PBMCs using ELISA.
Figure 10A: Inhibition of OVA-specific IgE expression by MTM bispecific antibody in a B-hIL4/IL4Rα humanized mouse asthma model;
Figure 10B: Inhibition of eosinophil expression by MTM bispecific antibody in B-hIL4/IL4Rα humanized mouse asthma model;
Figure 11A: Inhibition of IgE expression by MTM bispecific antibody in B-hTSLP/TSLPR/IL7Rα humanized mouse asthma model.
Figure 11B: Inhibition of eosinophil expression by MTM bispecific antibody in B-hTSLP/TSLPR/IL7Rα humanized mouse asthma model.

### DETAILED DESCRIPTION

The embodiments of the present invention will be illustrated in detail with the below examples. However, those skilled in the art will understand that the following examples are merely illustrative of the present invention and should not be construed as limiting the scope of the present invention.

The experimental methods in the examples of the present invention are carried out under conventional conditions, for example, as described in the *Antibodies: A Laboratory Manual* (Cold Spring Harbor), the *Molecular Cloning: A Laboratory Manual*, or under conditions recommended by the raw material or commercial manufacturers. All reagents were obtained from commercial sources.

In the present invention, the BMK1 serves as a positive control molecule (Dupilumab antibody), the sequence of which is derived from SEQ ID NO: 162 and SEQ ID NO: 164 of Patent Application No. CN200980143007.6. The preparation method is described in the literature (doi: 10.1016/j.ejbt.2019.07.002).

### Example 1: Preparation and Sequencing of Murine Anti-hIL-4Rα Antibodies

### 1.1 Animal Immunization

Approximately 10⁷ CHO-K1 cells stably overexpressing human IL-4Rα protein (Peprotech, 200-04R) were used as antigens. Four BALB/c mice were immunized by abdominal subcutaneous injection. After the initial immunization, booster immunizations were administered every two weeks. Following the second immunization, blood was collected from the tail vein, and mouse serum titers were determined by ELISA. Spleen cells from mice with titers greater than 2×10⁴ were used for subsequent hybridoma cell fusion.

### 1.2 Preparation of Hybridoma Cell Bank

Spleen cells from BALB/c mice with titers greater than 2×10⁴ were fused with SP2/0 mouse myeloma cells by electrofusion at a ratio of 1:1.2. The fused hybridoma cells were seeded into 96-well plates at a density of 1×10⁴ cells per well and cultured at 37°C for 10 days prior to screening.

### 1.3 Subcloning and cell-sorting, followed by preliminary screening of positive clones binding to hIL-4Rα by ELISA

Using the semi-solid medium method, the 30 screened hybridoma cell banks were subcloned cell-sorting to obtain monoclonal cells. Subsequently, positive clones binding to hIL-4Rα protein were further screened by ELISA.

Streptavidin (Jackson, Cat: 016-000-113) was diluted to a concentration of 0.25 µg/mL with coating buffer (200 mM Na₂CO₃/NaHCO₃, pH 9.2), mixed well, and added to a 384-well plate at 30 µL/well. The plate was coated overnight at 4°C and washed once with 100 µL/well of 1× PBS-T (0.05% Tween 20). Then, 80 µL of blocking buffer (2% BSA/1× PBS) was added to each well, incubated at 25°C for 1 hour, and washed three times with 100 µL/well of 1× PBS-T (0.05% Tween 20).IL-4Rα-biotin was diluted to 0.05 µg/mL with 2% BSA buffer (200 mM Na₂CO₃/NaHCO₃, pH 9.2), mixed well, and added to the 384-well plate at 30 µl/well, followed by washing three times with 100 µL/well of 1× PBS-T (0.05% Tween 20). Subsequently, 30 µL/well of the culture supernatant from the subcloned cells was added to the 384-well plate and incubated at 25°C for 2 hours, then washed three times with 100 µl/well of 1× PBS-T (0.05% Tween 20). Next, 30 µL of goat anti-mouse IgG Fc-HRP (diluted 1:5000) (Bethyl, Cat: A90-231P) was added to each well, incubated at 25°C for 1 hour, and washed six times with 100 µl/well of 1× PBS-T (0.05% Tween 20). Finally, 30 µL of TMB substrate was added to each well and allowed to react at 25°C for 20 minutes. The reaction was terminated by adding 30 µL of terminal solution (2 M HCl) to each well, and the absorbance was measured at a wavelength of 450 nm.

### 1.4 Further Screening of Positive Clones Binding to hIL-4Rα by Fluorescence-Activated Cell Sorting (FACS)

Based on the absorbance values obtained from ELISA, 60 molecules with the highest absorbance values were selected for FACS analysis (using CHO-K1.IL-4Rα cells). CHO-K1.IL-4Rα cells overexpressing hIL-4Rα were collected in a 50 mL centrifuge tube and added to a 96-well plate at a cell count of 1E5 cells per well. The plate was centrifuged at 1500 rpm for 4 minutes, and the supernatant was discarded. Then, 100 µL/well of the culture supernatant from the subcloned cells was added to the 96-well plate and incubated at 4°C for 1 hour, and washed twice with 200 µL/well of 1% BSA/1× PBS. 100 µL/well of PE-labeled goat anti-mouse IgG Fc (1:100) (Jackson, Cat: 115-115-164) was added, incubated at 4°C for 0.5 hours, and washed twice with 80 µl/well of 1% BSA/1× PBS. The cells were then resuspended in 30 µL/well of 1% BSA/1× PBS, and finally, the mean fluorescence intensity was measured by flow cytometry.

### 1.5 Screening of Positive Clones Capable of Inhibiting hIL-4 Activity Using a Reporter Gene Assay (HEK-293T.STAT6-GFP Cells)

Based on the ELISA results, 60 molecules with the highest absorbance values were selected for single-concentration reporter gene assay detection (using CHO-K1.IL-4Rα cells). HEK-293T.STAT6-GFP cells were prepared, resuspended in TransDetect BrightFluore DMEM + 10% FBS medium, and the cell density was adjusted to 5E5 cells/mL. The cell suspension was added to a black 96-well plate at 100 µL/well and incubated at 37°C for 24 hours. hIL-4 was diluted to a concentration of 1.0 ng/mL with TransDetect BrightFluore DMEM + 10% FBS medium, and 20 µl was added to each well. Subsequently, 80 µl of the subclone culture supernatant was added to the wells, mixed gently, and the incubation was continued at 37°C for another 24 hours. The fluorescence signal intensity was measured using a microplate reader with an excitation wavelength of 488 nm and an emission wavelength of 520 nm.

### 1.6 Expression, Purification, and Activity Evaluation of Candidate Molecules

Based on the results evaluated by the above three methods, the top 10 murine monoclonal cells were selected for scaled-up culture. The antibodies purified with Protein A chromatography from the culture supernatants were evaluated by FACS and reporter gene assays.

### 1.7 Screening of Molecules Binding to hIL-4Rα Protein by FACS

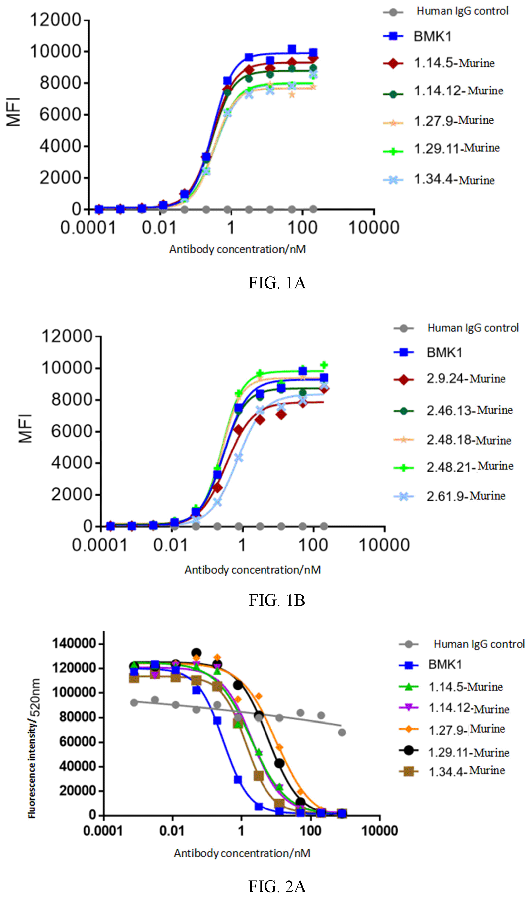

10 murine monoclonal antibody molecules with the best comprehensive evaluation results (see Table 1) were selected for complete FACS screening. CHO-K1.IL-4Rα cells overexpressing hIL-4Rα were collected in a 50 ml centrifuge tube. The cells were added to a 96-well plate at a density of 1E5 cells counts per well, centrifuged at 1500 rpm for 4 minutes, and the supernatant was discarded. Dilute the purified antibody to a concentration of 200 nM using 1% BSA / 1× PBS solution, followed by 11 serial 1:4 dilution. The diluted antibodies were incubated with the cells at 4°C for 1 hour, and washed twice with 200 µL/well of 1% BSA/1× PBS. Then, 100 µL/well of PE-labeled goat anti-mouse IgG Fc (1:100) was added, incubated at 4°C for 0.5 hours, and washed twice with 80 µL/well of 1% BSA/1× PBS. The cells were subsequently resuspended in 30 µL/well of 1% BSA/1× PBS. Finally, the mean fluorescence intensity was measured by flow cytometry, and the EC50 values were calculated using a four-parameter logistic curve fit. The results are shown in Table 1 and Figures 1A and 1B, with BMK1 serving as the positive control and human IgG4 ctrl as the negative control.

### 1.8 Screening of Molecules Blocking hIL-13 Activity by Reporter Gene Assay

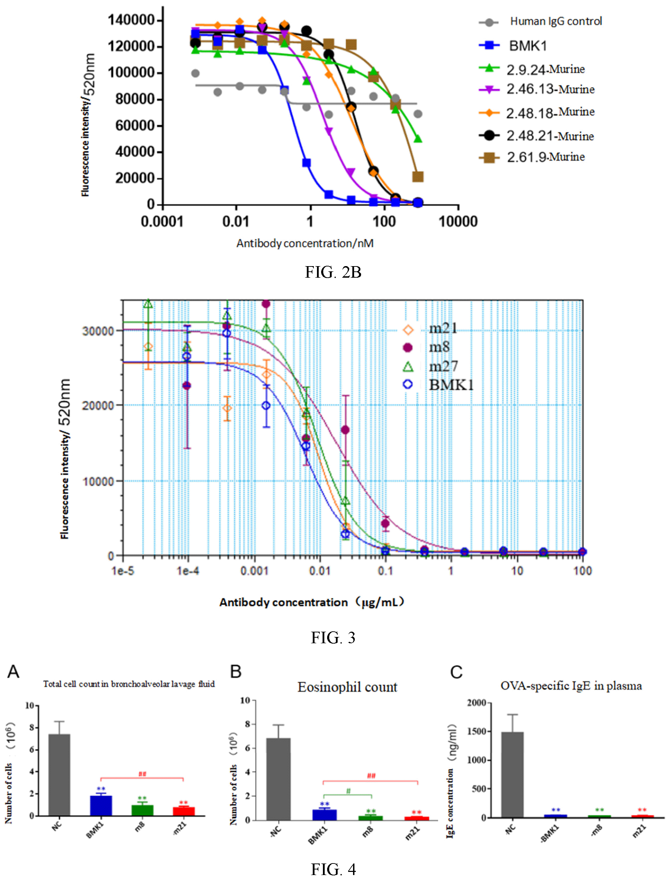

10 murine antibody molecules with the best comprehensive evaluation result (see Table 1) were selected for the complete reporter gene assay. HEK-293T.STAT6-GFP cells were resuspended in TransDetect BrightFluore DMEM + 10% FBS medium, and the cell density was adjusted to 5E5 cells/mL. The cell suspension was added to a black 96-well plate at 100 µL/well and incubated at 37°C for 24 hours. hIL-13 was diluted to a concentration of 20.0 ng/ml with TransDetect BrightFluore DMEM + 10% FBS medium. The antibodies were diluted with the pre-diluted hIL-13 solution with 11 serilal 1:4 dilutions. The serial solutions were added into a 96-well plate with 100 µL/well followed by gently mixing. The plate was incubated at 37°C for 24 hours. The fluorescence signal intensity was measured using a microplate reader with an excitation wavelength of 488 nm and an emission wavelength of 520 nm. The IC50 values were calculated by fitting a four-parameter logistic curve. The results are shown in Table 1 and Figures 2A and 2B, with BMK1 serving as the positive control and human IgG4 ctrl as the negative control.

### 1.9 Affinity Detection

Based on the comprehensive analysis of the above data, 2 molecules with the best activity were selected for affinity detection. Real-time biosensor surface plasmon resonance analysis (Cytiva, Biacore 8K) was performed to determine the binding affinity (KD) of the 2 antibody molecules to hIL-4Rα at 25°C. Firstly, anti-mouse Fc IgG antibody was coupled to a CM5 chip to form an antibody capture surface. Subsequently, the 2 selected antibody molecules were further coupled to the anti-mouse Fc IgG antibody at a flow rate of 10 µl/min for 30 seconds. The analyte (hIL-4Rα.ECD) was prepared into 6 serial 1:2 dilutions and detected sequentially, with an association time of 180 seconds and a dissociation time of 1800 seconds at a flow rate of 10 µl/min. Finally, kinetic analysis was performed using BIA evaluation software to determine the equilibrium association constant (Ka), equilibrium dissociation constant (Kd), and affinity constant (KD). The detection results are shown in Table 1, indicating that both molecules exhibited relatively high affinity to hIL-4Rα.

**Table 1: Activity and Affinity Data for Molecules**

| Molecules | Cell-based Assay Data (hIL-13) | FACS Data (CHO-K1-hIL-4Rα) | |
|---|---|---|---|
| | IC50 (nM) | EC50 (nM) | |
| 1.14.5-Murine | 1.86 | 0.29 | |
| 1.14.12-Murine | 2.02 | 0.28 | |
| 1.27.9-Murine | 10.22 | 0.32 | |
| 1.29.11-Murine | 6.04 | 0.35 | |
| 1.34.4-Murine | 1.38 | 0.35 | |
| 2.9.24-Murine | NA | 0.35 | |
| 2.46.13-Murine | 2.23 | 0.28 | |
| 2.48.18-Murine | 12.72 | 0.26 | |
| 2.48.21-Murine | 15.52 | 0.27 | |
| 2.61.9-Murine | NA | 0.71 | |
| BMK1 | 0.81 | 0.30 | |

| Affinity Data(hIL-4Rα) | | | |
|---|---|---|---|
| Molecules | Ka (1/Ms) | Kd (1/s) | KD (M) |
| 1.34.4-Murine | 3.61E+05 | 1.33E-04 | 3.69E-10 |
| 2.46.13-Murine | 3.85E+05 | 1.18E-04 | 3.06E-10 |

### 1.10 Sequencing of the variable region sequences of the candidate murine antibody

The 2 murine monoclonal antibody molecules mentioned above (1.34.4-murine and 2.46.13-murine) were sequenced to confirm and analyze their sequences. The sequence information is shown in Table 2.

**Table 2. List of CDR Sequences of Murine Monoclonal Antibody Molecules**

| Molecules | Name | Sequences | Number |
|---|---|---|---|
| 1.34.4-Murine | HCDR1 | GYTFTTYYIY | SEQ ID NO: 1 |
| | HCDR2 | GSNPSTGGTLFNEKFKT | SEQ ID NO: 10 |
| | HCDR3 | SGRYAPFTY | SEQ ID NO: 3 |
| | LCDR1 | KASQDVSTSVA | SEQ ID NO: 4 |
| | LCDR2 | SASYRFT | SEQ ID NO: 5 |
| | LCDR3 | QQYSTTPFT | SEQ ID NO: 11 |
| 2.46.13-Murine | HCDR1 | GFTFSSYYMS | SEQ ID NO: 12 |
| | HCDR2 | AINSNGGSTYSPDTVKG | SEQ ID NO: 13 |
| | HCDR3 | RSLLGNHRDWYFDV | SEQ ID NO: 14 |
| | LCDR1 | RASENIYSYLA | SEQ ID NO: 15 |
| | LCDR2 | NAKTLAD | SEQ ID NO: 16 |
| | LCDR3 | LHHYGTPLT | SEQ ID NO: 17 |

The variable region of heavy chain sequence of the 1.34.4-murine monoclonal antibody molecule is:

QVQLQQPGAELVKPGASMKLSCKASGYTFTTYYIYWVKQRPGQGLEWIGGSNPS
TGGTLFNEKFKTKATLTVDKYSSTAYMELSSLTFEDSAVYFCTRSGRYAPFTYWGQGTLV
TVSA(SEQ ID NO: 18)

The variable region of light chain sequence of the 1.34.4-murine monoclonal antibody molecule is:

DIVMTQSHKVLSTSVGDRVIITCKASQDVSTSVAWYQQRPGHSPKLLIYSASYRFT

GVPDRFTGSGSGTDFTFTISSVQAEDLAVYNCQQYSTTPFTFGSGTKLEIK(SEQ ID NO:
19)

The variable region of heavy chain sequence of the 2.46.13-murine monoclonal antibody molecule is:

DVKLVESGGGLVKLGGSLKLSCAASGFTFSSYYMSWVRQTPEKRLELVAAINSNG
GSTYSPDTVKGRFTISRDNAKNTLYLQMSSLKSEDTALYYCARRSLLGNHRDWYFDVW
GAGTTVTVSS(SEQ ID NO: 20)

The variable region of light chain sequence of the 2.46.13-murine monoclonal antibody molecule is:

DIQMTQSPASLSASVGETVTITCRASENIYSYLAWYQQKQGKSPQLLVYNAKTLAD
GVPSRFSGSGSGTQFSLKINTLQPEDFGSYYCLHHYGTPLTFGAGTKLELK(SEQ ID NO:
21)

### Example 2: Antibody Humanization and Affinity Evaluation

The variable region sequences of the two murine monoclonal antibodies, 1.34.4-murine and 2.46.13-murine, were aligned with human germline antibody sequences to identify sequences with high homology for CDR grafting. The heavy chain CDR regions of 1.34.4-murine were grafted into the framework regions of IGHV1-46*01 + IGHJ1*01, and the light chain CDR regions were grafted into the framework regions of IGKV1-39*01 + IGKJ2*01. The heavy chain CDR regions of 2.46.13-murine were grafted into the framework regions of IGHV3-21*01 + IGHJ6*01, and the light chain CDR regions were grafted into the framework regions of IGKV1-39*01 + IGKJ2*01. The sequences are shown in Tables 3-1 and 3-2. Subsequently, homology modeling was performed using computer software to analyze the amino acid sequences of the CDR regions and their framework regions. Key amino acid sites potentially involved in interacting with the hIL-4Rα protein and stabilizing the spatial structure were predicted. Based on this analysis, back-mutation sites were designed and screened. The humanized variable region sequences incorporating the designed back-mutations were linked to the human IgG4 constant region, followed by antibody expression (expression method referenced from doi: 10.1016/j.ejbt.2019.07.002) and affinity evaluation (method same as 1.9). Based on the affinity data, the 1.34.4-z18 humanized antibody molecule, which underwent back-mutation, was ultimately selected for affinity maturation. The sequences of 1.34.4-z18 are shown in Tables 3-1 and 3-2, and the affinity data are presented in Table 4.

**Table 3-1. List of CDR Sequences of Humanized Monoclonal Antibody Molecules**

| Molecules | Name | Sequences | Number |
|---|---|---|---|
| 1.34.4 | HCDR1 | GYTFTTYYIY | SEQ ID NO: 1 |
| | HCDR2 | GSNPSTGGTLFNEKFKT | SEQ ID NO: 10 |
| | HCDR3 | SGRYAPFTY | SEQ ID NO: 3 |
| | LCDR1 | KASQDVSTSVA | SEQ ID NO: 4 |
| | LCDR2 | SASYRFT | SEQ ID NO: 5 |
| | LCDR3 | QQYSTTPFT | SEQ ID NO: 11 |
| 2.46.13 | HCDR1 | GFTFSSYYMS | SEQ ID NO: 12 |
| | HCDR2 | AINSNGGSTYSPDTVKG | SEQ ID NO: 13 |
| | HCDR3 | RSLLGNHRDWYFDV | SEQ ID NO: 14 |
| | LCDR1 | RASENIYSYLA | SEQ ID NO: 15 |
| | LCDR2 | NAKTLAD | SEQ ID NO: 16 |
| | LCDR3 | LHHYGTPLT | SEQ ID NO: 17 |
| 1.34.4-z18 | HCDR1 | GYTFTTYYIY | SEQ ID NO: 1 |
| | HCDR2 | GSNPSTGGTLFNEKFKT | SEQ ID NO: 10 |
| | HCDR3 | SGRYAPFTY | SEQ ID NO: 3 |
| | LCDR1 | KASQDVSTSVA | SEQ ID NO: 4 |
| | LCDR2 | SASYRFT | SEQ ID NO: 5 |
| | LCDR3 | QQYSTTPFT | SEQ ID NO: 11 |

**Table 3-2. List of Variable Region Sequences of Humanized Monoclonal Antibody Molecules**

| Molec ules | Light/Heavy Chain Variable Regions | Sequences | Number |
|---|---|---|---|
| 1.34.4 | variable region of heavy chain | QVQLVQSGAEVKKPGASVKVSCKASGYTFTTYYIYW VRQAPGQGLEWMGGSNPSTGGTLFNEKFKTRVTMT RDTSTSTVYMELSSLRSEDTAVYYCARSGRYAPFTYW GQGTLVTVSS | SEQ ID NO: 22 |
| | variable region of light chain | DIQMTQSPSSLSASVGDRVTITCKASQDVSTSVAWYQ QKPGKAPKLLIYSASYRFTGVPSRFSGSGSGTDFTLTI SSLQPEDFATYYCQQYSTTPFTFGQGTKLEIK | SEQ ID NO: 23 |
| 2.46.1 3 | variable region of heavy chain | EVQLVESGGGLVKPGGSLRLSCAASGFTFSSYYMSW VRQAPGKGLEWVSAINSNGGSTYSPDTVKGRFTISRD NAKNSLYLQMNSLRAEDTAVYYCARRSLLGNHRDW YFDVWGQGTTVTVSS | SEQ ID NO: 24 |
| | variable region of light chain | DIQMTQSPSSLSASVGDRVTITCRASENIYSYLAWYQ QKPGKAPKLLIYNAKTLADGVPSRFSGSGSGTDFTLTI SSLQPEDFATYYCLHHYGTPLTFGQGTKLEIK | SEQ ID NO: 25 |
| 1.34.4 -z18 | variable region of heavy chain | QVQLVQSGAEVKKPGASVKVSCKASGYTFTTYYIYW VRQAPGQGLEWMGGSNPSTGGTLFNEKFKTRVTMT VDKSTSTAYMELSSLRSEDTAVYYCTRSGRYAPFTYW GQGTLVTVSS | SEQ ID NO: 26 |
| | variable region of light chain | DIQMTQSPSSLSASVGDRVTITCKASQDVSTSVAWYQ QKPGKAPKLLIYSASYRFTGVPSRFSGSGSGTDFTFTI SSLQPEDFATYYCQQYSTTPFTFGQGTKLEIK | SEQ ID NO: 27 |

**Table 4. Affinity Data of Humanized Antibody Molecules**

| Molecules | Ka (1/Ms) | Kd (1/s) | KD (M) |
|---|---|---|---|
| 1.34.4 | 1.96E+05 | 1.00E-04 | 5.12E-10 |
| 1.34.4-z18 | 2.42E+05 | 1.37E-04 | 5.67E-10 |

### Example 3: Affinity Maturation

### 3.1 Single-Site Saturation Mutagenesis

Using saturation mutagenesis, each CDR site of the 1.34.4-z18 humanized antibody molecule was subjected to saturation mutagenesis to establish a Fab single-site mutation library (>5000 molecules). The binding activity of the Fab single-site mutation library was screened using competitive ELISA. Top 15 Fab positive clones identified through screening were subjected to single-point affinity detection and sequencing. Mutants with a 2 to 10-fold reduction in dissociation constant were selected for subsequent combinatorial mutagenesis.

### 3.2 Combinatorial Mutation Molecular Affinity Screening

After identifying the sites with the most significant improvement in affinity, combinatorial mutation screening was performed on these sites. The dissociation constants of the resulting molecules were all less than 1.00E-05 (1/s), which was below the detection limit of the instrument, and the KD were all less than 9.43E-11 (M), indicating that all affinity-matured molecules could bind to the hIL-4Rα protein with high affinity. Ultimately, the m8, m21, and m27 molecules were selected for evaluation of cellular activity and affinity. The sequence information is shown in Table 5.

**Table 5. CDR Sequences of Monoclonal Antibody Molecules after Affinity Maturation**

| Molecules | Name | Sequences | Number |
|---|---|---|---|
| m8 | HCDR1 | GYTFTTYYIY | SEQ ID NO: 1 |
| | HCDR2 | GSNPSTGGTLDNEKFKT | SEQ ID NO: 2 |
| | HCDR3 | SGRYAPFTY | SEQ ID NO: 3 |
| | LCDR1 | KASQDVSTSVA | SEQ ID NO: 4 |
| | LCDR2 | SASYRFT | SEQ ID NO: 5 |
| | LCDR3 | QHYSTTPFT | SEQ ID NO: 6 |
| m21 | HCDR1 | GYTFTTYYIY | SEQ ID NO: 1 |
| | HCDR2 | GSNPSTGGTLENEKFKT | SEQ ID NO: 7 |
| | HCDR3 | SGRYAPFTY | SEQ ID NO: 3 |
| | LCDR1 | KASQDVSTSVA | SEQ ID NO: 4 |
| | LCDR2 | SASYRFT | SEQ ID NO: 5 |
| | LCDR3 | VHYSTTPFT | SEQ ID NO: 8 |
| m27 | HCDR1 | GYTFTTYYIY | SEQ ID NO: 1 |
| | HCDR2 | GSNPSTGGTLENEKFKT | SEQ ID NO: 7 |
| | HCDR3 | SGRYATFTY | SEQ ID NO: 9 |
| | LCDR1 | KASQDVSTSVA | SEQ ID NO: 4 |
| | LCDR2 | SASYRFT | SEQ ID NO: 5 |
| | LCDR3 | VHYSTTPFT | SEQ ID NO: 8 |

The variable region of heavy chain sequence of the m8 molecule after affinity maturation is:

QVQLVQSGAEVKKPGASVKVSCKASGYTFTTYYIYWVRQAPGQGLEWMGGSNPS
TGGTLDNEKFKTRVTMTVDKSTSTAYMELSSLRSEDTAVYYCTRSGRYAPFTYWGQGTL
VTVSS(SEQ ID NO: 28)

The variable region of light chain sequence of the m8 molecule after affinity maturation is:

DIQMTQSPSSLSASVGDRVTITCKASQDVSTSVAWYQQKPGKAPKLLIYSASYRFTG
VPSRFSGSGSGTDFTFTISSLQPEDFATYYCQHYSTTPFTFGQGTKLEIK(SEQ ID NO: 29)

The variable region of heavy chain sequence of the m21 molecule after affinity maturation is:

QVQLVQSGAEVKKPGASVKVSCKASGYTFTTYYIYWVRQAPGQGLEWMGGSNPS
TGGTLENEKFKTRVTMTVDKSTSTAYMELSSLRSEDTAVYYCTRSGRYAPFTYWGQGTL
VTVSS(SEQ ID NO: 30)

The variable region of light chain sequence of the m21 molecule after affinity maturation is:

DIQMTQSPSSLSASVGDRVTITCKASQDVSTSVAWYQQKPGKAPKLLIYSASYRFTG
VPSRFSGSGSGTDFTFTISSLQPEDFATYYCVHYSTTPFTFGQGTKLEIK(SEQ ID NO: 31)

The variable region of heavy chain sequence of the m27 molecule after affinity maturation is:

QVQLVQSGAEVKKPGASVKVSCKASGYTFTTYYIYWVRQAPGQGLEWMGGSNP
STGGTLENEKFKTRVTMTVDKSTSTAYMELSSLRSEDTAVYYCTRSGRYATFTYWGQGT
LVTVSS(SEQ ID NO: 32)

The variable region of light chain sequence of the m27 molecule after affinity maturation is:

DIQMTQSPSSLSASVGDRVTITCKASQDVSTSVAWYQQKPGKAPKLLIYSASYRFTG
VPSRFSGSGSGTDFTFTISSLQPEDFATYYCVHYSTTPFTFGQGTKLEIK(SEQ ID NO: 33)

### 3.3 Detection of Antibody Molecules Blocking the Binding of hIL-4 to hIL-4Rα Protein Using Reporter Gene Assay

The reporter gene assay was employed for further cellular activity detection of the aforementioned molecules. HEK-293T.STAT6-GFP cells were resuspended in TransDetect BrightFluore DMEM + 10% FBS medium, and the cell density was adjusted to 5E5 cells/mL. The cells were added to black 96-well plates at 100 µL/well and incubated at 37°C for 24 hours. hIL-4 was diluted to a concentration of 1.0 ng/ml using TransDetect BrightFluore DMEM + 10% FBS medium. Dilute the antibody using the pre-diluted hIL-4 solution, with 11 serial 1:4 dilutions. Then, add 100 µL/well to a 96-well plate, mixed gently, and incubated at 37°C for another 24 hours. The fluorescence signal intensity was measured using a microplate reader with an excitation wavelength of 488 nm and an emission wavelength of 520 nm. A four-parameter curve fitting was applied, yielding the results shown in Figure 3 and Table 6. BMK1 was used as a positive control, and all molecules demonstrated significant inhibition of hIL-4 activity.

**Table 6. Detection of hIL-4 Blocking Activity by Reporter Gene Assay**

| Molecules | IC50(pM) |
|---|---|
| m8 | 16.4 |
| m21 | 286 |
| m27 | 249 |
| BMK1 | 201 |

### 3.4 Detection of the Affinity of Candidate Molecules to the hIL-4Rα Protein Using Surface Plasmon Resonance

First, anti-human Fc IgG antibody was coupled to a CM5 chip to form an antibody capture surface. Subsequently, the selected antibody molecules were further coupled to the anti-human Fc IgG antibody at a flow rate of 10 µl/min for 30 seconds. The analyte (hIL-4Rα.ECD) was prepared into 6 serial 1:2 dilution gradients and detected sequentially, with an association time of 180 seconds and a dissociation time of 1800 seconds at a flow rate of 10 µl/min. Finally, kinetic analysis was performed using BIA evaluation software to determine the equilibrium association constant (Ka), equilibrium dissociation constant (Kd), and affinity constant (KD). The detection results are shown in Table 7. BMK1 was used as a positive control molecule, and the candidate molecules exhibited affinity comparable to that of the control molecule.

**Table 7. Affinity of Candidate Molecules for hIL-4Rα Protein Detected by Surface Plasmon Resonance**

| Molecules | Ka (1/Ms) | Kd (1/s) | KD (M) |
|---|---|---|---|
| m8 | 9.03E+05 | 3.95E-05 | 4.37E-11 |
| m21 | 5.82E+05 | 7.76E-05 | 1.33E-10 |
| m27 | 5.68E+05 | 1.12E-04 | 1.96E-10 |
| BMK1 | 1.43E+06 | 5.40E-05 | 3.79E-11 |

### 3.5 Evaluation of the Inhibitory Activity of Candidate Molecules on hIL-13 Using TF-1-based Cell Proliferation Assay

The inhibitory activity of m8, m21, and m27 was further evaluated using a proliferation inhibition assay. Adjust the density of TF-1 cells to 5E5 cells/mL using RPMI 1640 medium supplemented with 5% FBS. The cells were added to white 96-well plates at 100 µL/well. hIL-13 was diluted to a concentration of 30 ng/ml using the culture medium. Dilute the antibody using the pre-diluted hIL-13 solution, with 11 serial 1:4 dilutions. Then, add 50 µL/well to a 96-well plate, mixed gently, and incubated at 37°C for another 72 hours. The 96-well plate was removed, and 100 µL/well of CellTiter-Glo color reagent was added, followed by incubation at room temperature for 15 minutes. Luminescence intensity was measured using a microplate reader, and the results are shown in Table 8. BMK1 was used as a positive control. The m21 and m27 antibody molecules exhibited superior inhibitory activity compared to BMK1, while m8 antibody molecule demonstrated blocking activity comparable to that of BMK1.

**Table 8. hIL-13 Blocking Activity Data Detected by Proliferation Inhibition Assay**

| Molecules | IC50(pM) |
|---|---|
| m8 | 125 |
| m21 | 66.5 |
| m27 | 58.8 |
| BMK1 | 140 |

### Example 4: Effects of m8 and m21 on OVA-Induced Asthma Model in B-hIL4/IL-4Rα Mice

An OVA (ovalbumin)-induced asthma model was established in B-hIL4/IL-4Rα mice. D0 (Day 0) marked the first day of model establishment. On D0 and D14, mice were sensitized by intraperitoneal injection of 10 µg OVA. From D21 to D24, mice were challenged intranasally with 5% OVA. The experimental mice were divided into four groups (8 mice per group), including a negative control group (NC), a positive control group (BMK1), and experimental groups (m8 and m21). The administered dose was 25 mg/kg via subcutaneous injection on D7, D10, D14, D17, D21, and D24. During the period, body weight changes of mice in each group were recorded. On D25, blood, bronchoalveolar lavage fluid (BALF), and lung tissues were collected from mice in each group for indicator assessment.

The experimental results are shown in Figures 4A and 4B. Compared with the NC control group, the numbers of BALF-derived lymphocytes and eosinophils in the experimental groups and the positive control group were significantly reduced, with m8 and m21 showing greater reductions. Meanwhile, the OVA-specific IgE levels in plasma were measured, as shown in Figure 4C. Compared with the NC control group, the OVA-specific IgE levels in the experimental groups and the positive control group were significantly reduced.

### Example 5: Analysis of Molecular Physicochemical Properties

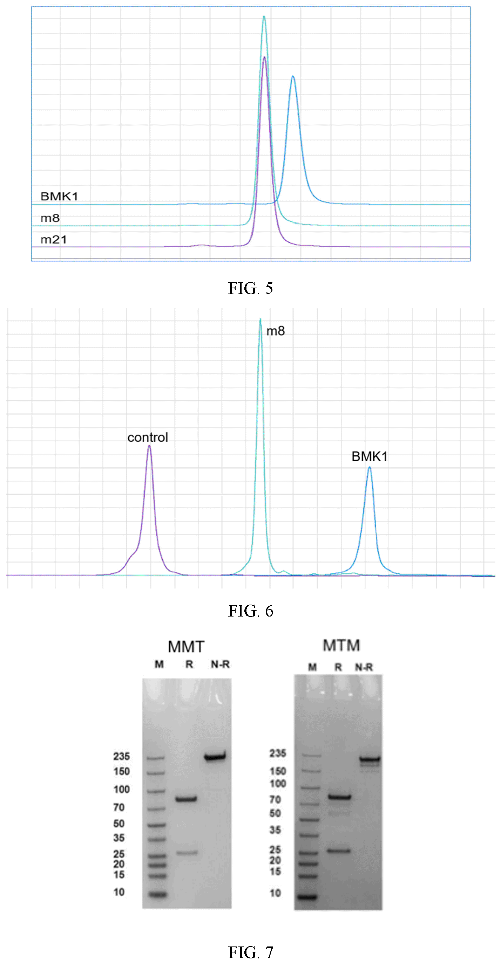

The purity of samples obtained after one-step Protein A purification was assessed using molecular exclusion chromatography. The results, indicated the monomer content for all samples was greater than 98%, shown in Figure 5, demonstrating that the molecules exhibited good stability during expression and purification, and were not prone to aggregate formation. The hydrophobicity of the m8 molecule was evaluated using hydrophobic interaction chromatography, and the results were shown in Figure 6. The control, a low-hydrophobicity antibody molecule, peaks eluted rapidly with a shorter retention time. The retention time of the m8 molecule was significantly lower than that of the positive antibody BMK1, indicating that its hydrophobicity was significantly lower than that of the positive antibody BMK1. This characteristic offers significant advantages for subsequent process development.

### Example 6: Preparation of the Anti-TSLP Antibody Tezepelumab

The sequence of the marketed anti-TSLP monoclonal antibody Tezepelumab was obtained from Chinese published patent CN101809035A, and the nucleic acid sequence was synthesized by GenScript.

The amino acid sequence of the Tezepelumab variable region of heavy chain (TezeHv) is:

QMQLVESGGGVVQPGRSLRLSCAASGFTFRTYGMHWVRQAPGKGLEWVAVIWYD
GSNKHYADSVKGRFTITRDNSKNTLNLQMNSLRAEDTAVYYCARAPQWELVHEAFDIW
GQGTMVTVSS(SEQ ID NO: 34)

The amino acid sequence of the Tezepelumab constant region of heavy chain is:

ASTKGPSVFPLAPCSRSTSESTAALGCLVKDYFPEPVTVSWNSGALTSGVHTFPAVL
QSSGLYSLSSVVTVPSSNFGTQTYTCNVDHKPSNTKVDKTVERKCCVECPPCPAPPVAGP
SVFLFPPKPKDTLMISRTPEVTCVVVDVSHEDPEVQFNWYVDGVEVHNAKTKPREEQF
NSTFRVVSVLTVVHQDWLNGKEYKCKVSNKGLPAPIEKTISKTKGQPREPQVYTLPPSRE
EMTKNQVSLTCLVKGFYPSDIAVEWESNGQPENNYKTTPPMLDSDGSFFLYSKLTVDKS
RWQQGNVFSCSVMHEALHNHYTQKSLSLSPGK(SEQ ID NO: 35)

The amino acid sequence of the Tezepelumab variable region of light chain (TezeLv) is:

SYVLTQPPSVSVAPGQTARITCGGNNLGSKSVHWYQQKPGQAPVLVVYDDSDRPS
WIPERFSGSNSGNTATLTISRGEAGDEADYYCQVWDSSSDHVVFGGGTKLTVL(SEQ ID
NO: 36)

The amino acid sequence of the Tezepelumab constant region of light chain is:

GQPKAAPSVTLFPPSSEELQANKATLVCLISDFYPGAVTVAWKADSSPVKAGVETTT
PSKQSNNKYAASSYLSLTPEQWKSHRSYSCQVTHEGSTVEKTVAPTECS(SEQ ID NO: 37)

The amino acid sequence of the Tezepelumab heavy chain (Tezepelumab-HC) is:

QMQLVESGGGVVQPGRSLRLSCAASGFTFRTYGMHWVRQAPGKGLEWVAVIWYDGSN
KHYADSVKGRFTITRDNSKNTLNLQMNSLRAEDTAVYYCARAPQWELVHEAFDIWGQG
TMVTVSSASTKGPSVFPLAPCSRSTSESTAALGCLVKDYFPEPVTVSWNSGALTSGVHTF
PAVLQSSGLYSLSSVVTVPSSNFGTQTYTCNVDHKPSNTKVDKTVERKCCVECPPCPAPP
VAGPSVFLFPPKPKDTLMISRTPEVTCVVVDVSHEDPEVQFNWYVDGVEVHNAKTKPRE
EQFNSTFRVVSVLTVVHQDWLNGKEYKCKVSNKGLPAPIEKTISKTKGQPREPQVYTLP
PSREEMTKNQVSLTCLVKGFYPSDIAVEWESNGQPENNYKTTPPMLDSDGSFFLYSKLTV

DKSRWQQGNVFSCSVMHEALHNHYTQKSLSLSPGK(SEQ ID NO: 53)

The amino acid sequence of the Tezepelumab light chain (Tezepelumab-LC) is:

SYVLTQPPSVSVAPGQTARITCGGNNLGSKSVHWYQQKPGQAPVLVVYDDSDRPSWIPE
RFSGSNSGNTATLTISRGEAGDEADYYCQVWDSSSDHVVFGGGTKLTVLGQPKAAPSVT
LFPPSSEELQANKATLVCLISDFYPGAVTVAWKADSSPVKAGVETTTPSKQSNNKYAASS
YLSLTPEQWKSHRSYSCQVTHEGSTVEKTVAPTECS(SEQ ID NO: 54)

The cDNA sequences of the Tezepelumab heavy chain and light chain were cloned into the pKS001 vector, respectively, to obtain recombinant expression plasmids for Tezepelumab expression. The recombinant plasmids were transiently transfected into ExpiCHOS cells (Thermo Fisher Scientific), and Tezepelumab was purified from the culture supernatant using Protein A affinity chromatography.

### Example 7: Preparation of the Anti-IL-4Rα Antibody Dupilumab

The amino acid sequence of the Dupilumab heavy chain (Dup-HC) is:

EVQLVESGGGLEQPGGSLRLSCAGSGFTFRDYAMTWVRQAPGKGLEWVSSISGSG
GNTYYADSVKGRFTISRDNSKNTLYLQMNSLRAEDTAVYYCAKDRLSITIRPRYYGLDV
WGQGTTVTVSSASTKGPSVFPLAPCSRSTSESTAALGCLVKDYFPEPVTVSWNSGALTSG
VHTFPAVLQSSGLYSLSSVVTVPSSSLGTKTYTCNVDHKPSNTKVDKRVESKYGPPCPPC
PAPEFLGGPSVFLFPPKPKDTLMISRTPEVTCVVVDVSQEDPEVQFNWYVDGVEVHNAK
TKPREEQFNSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGSFFLY
SRLTVDKSRWQEGNVFSCSVMHEALHNHYTQKSLSLSLGK(SEQ ID NO: 38)

The amino acid sequence of the Dupilumab light chain (Dup-LC) is:

DIVMTQSPLSLPVTPGEPASISCRSSQSLLYSIGYNYLDWYLQKSGQSPQLLIYLGSN
RASGVPDRFSGSGSGTDFTLKISRVEAEDVGFYYCMQALQTPYTFGQGTKLEIKRTVAA
PSVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC(SEQ ID NO: 39)

The cDNA sequences of the Dupilumab heavy chain and light chain were cloned into the pKS001 vector, respectively, to obtain recombinant expression plasmids for Dupilumab expression. The recombinant plasmids were transiently transfected into ExpiCHOS cells (Thermo Fisher Scientific), and Dupilumab was purified from the culture supernatant using Protein A affinity chromatography.

### Example 8: Preparation of the Anti-IL-4R Antibody m8

The amino acid sequence of the m8 molecule variable region of heavy chain (m8Hv) is:

QVQLVQSGAEVKKPGASVKVSCKASGYTFTTYYIYWVRQAPGQGLEWMGGSNPS

TGGTLDNEKFKTRVTMTVDKSTSTAYMELSSLRSEDTAVYYCTRSGRYAPFTYWGQGTL
VTVSS(SEQ ID NO: 28)

The amino acid sequence of the m8 molecule heavy chain constant region is:

ASTKGPSVFPLAPCSRSTSESTAALGCLVKDYFPEPVTVSWNSGALTSGVHTFPAVLQSSG
LYSLSSVVTVPSSSLGTKTYTCNVDHKPSNTKVDKRVESKYGPPCPPCPAPEFLGGPSVF
LFPPKPKDTLMISRTPEVTCVVVDVSQEDPEVQFNWYVDGVEVHNAKTKPREEQFNST
YRVVSVLTVLHQDWLNGKEYKCKVSNKGLPSSIEKTISKAKGQPREPQVYTLPPSQEEM
TKNQVSLTCLVKGFYPSDIAVEWESNGQPENNYKTTPPVLDSDGSFFLYSRLTVDKSRW
QEGNVFSCSVMHEALHNHYTQKSLSLSLGK(SEQ ID NO: 40)

The amino acid sequence of the m8 molecule variable region of light chain (m8Lv) is:

DIQMTQSPSSLSASVGDRVTITCKASQDVSTSVAWYQQKPGKAPKLLIYSASYRFTGVPS
RFSGSGSGTDFTFTISSLQPEDFATYYCQHYSTTPFTFGQGTKLEIK(SEQ ID NO: 29)

The amino acid sequence of the m8 molecule light chain constant region is:


(SEQ ID NO:41)

The amino acid sequence of the m8 molecule heavy chain (m8-HC) is:

QVQLVQSGAEVKKPGASVKVSCKASGYTFTTYYIYWVRQAPGQGLEWMGGSNPSTGG
TLDNEKFKTRVTMTVDKSTSTAYMELSSLRSEDTAVYYCTRSGRYAPFTYWGQGTLVTV
SSASTKGPSVFPLAPCSRSTSESTAALGCLVKDYFPEPVTVSWNSGALTSGVHTFPAVLQS
SGLYSLSSVVTVPSSSLGTKTYTCNVDHKPSNTKVDKRVESKYGPPCPPCPAPEFLGGPS
VFLFPPKPKDTLMISRTPEVTCVVVDVSQEDPEVQFNWYVDGVEVHNAKTKPREEQFN
STYRVVSVLTVLHQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFFLYSRLTVDKSR
WQEGNVFSCSVMHEALHNHYTQKSLSLSLGK(SEQ ID NO: 55)

The amino acid sequence of the m8 molecule light chain (m8-LC) is:

DIQMTQSPSSLSASVGDRVTITCKASQDVSTSVAWYQQKPGKAPKLLIYSASYRFTGVPS
RFSGSGSGTDFTFTISSLQPEDFATYYCQHYSTTPFTFGQGTKLEIKRTVAAPSVFIFPPSDE
QLKSGTASVVCLLNNFYPREAKVQWKVDNALQSGNSQESVTEQDSKDSTYSLSSTLTLS
KADYEKHKVYACEVTHQGLSSPVTKSFNRGEC(SEQ ID NO: 56)

The cDNA sequences of the m8 heavy chain and light chain were cloned into the pKS001 vector, respectively, to obtain recombinant expression plasmids for m8 expression. The recombinant plasmids were transiently transfected into ExpiCHOS cells (Thermo Fisher Scientific), and m8 was purified from the culture supernatant using Protein A affinity chromatography.

### Example 9: Sequence Design of the Bispecific Antibody

### 1. Sequence Design

The bispecific antibody structure in the present invention adopts the IgG-scFv format, where the scFv fragment of one antibody is linked to the C-terminus of both heavy chains of an intact IgG antibody. The main composition design of its heavy and light chains is shown in Table 9-2.

Based on considerations of molecular stability and activity, mutations were introduced into the light chain and heavy chain variable regions of the Tezepelumab and m8 molecules. The sequence information is as follows:

The mutated sequence of the variable region of heavy chain of Tezepelumab (MtezeHv) is:

QMQLVESGGGVVQPGRSLRLSCAASGFTFRTYGMHWVRQAPGKCLEWVAVIWYD
GSNKHYADSVKGRFTITRDNSKNTLNLQMNSLRAEDTAVYYCARAPQWELVHEAFDIW
GQGTMVTVSS; (SEQ ID NO: 42)

The mutated sequence of the variable region of light chain of Tezepelumab (MtezeLv) is:

SYVLTQPPSVSVAPGQTARITCGGNNLGSKSVHWYQQKPGQAPVLVVYDDSDRPS
WIPERFSGSNSGNTATLTISRGEAGDEADYYCQVWDSSSDHVVFGCGTKLTVL; (SEQ ID
NO: 43)

The mutated sequence of the variable region of heavy chain of m8 (Mm8Hv) is:

QVQLVQSGAEVKKPGASVKVSCKASGYTFTTYYIYWVRQAPGQCLEWMGGSNPS
TGGTLDNEKFKTRVTMTVDKSTSTAYMELSSLRSEDTAVYYCTRSGRYAPFTYWGQGTL
VTVSS; (SEQ ID NO: 44)

The mutated sequence of the variable region of light chain of m8 (Mm8Lv) is:

DIQMTQSPSSLSASVGDRVTITCKASQDVSTSVAWYQQKPGKAPKLLIYSASYRFTG
VPSRFSGSGSGTDFTFTISSLQPEDFATYYCQHYSTTPFTFGCGTKLEIK; (SEQ ID NO: 45)

The amino acid sequence of Linker1 is GGGGSGGGGS; (SEQ ID NO: 46)

The amino acid sequence of Linker2 is GGGGSGGGGSGGGGSGGGGS; (SEQ ID NO: 47)

**Table 9-1 The amino acid sequences of the complementarity determining regions of the immunoglobulin portion of MTM and MMT**

| Molecules | Name | Sequences | Number |
|---|---|---|---|
| MTM | HCDR1 | GFTFRTYG | SEQ ID NO: 48 |
| | HCDR2 | IWYDGSNK | SEQ ID NO: 49 |
| | HCDR3 | ARAPQWELVHEAFD | SEQ ID NO: 50 |
| | LCDR1 | NLGSKS | SEQ ID NO: 51 |
| | LCDR2 | DDS | / |
| | LCDR3 | QVWDSSSDHVV | SEQ ID NO: 52 |
| | HCDR1 | GYTFTTYYIY | SEQ ID NO: 1 |
| | HCDR2 | GSNPSTGGTLDNEKFKT | SEQ ID NO: 2 |
| MMT | HCDR3 | SGRYAPFTY | SEQ ID NO: 3 |
| | LCDR1 | KASQDVSTSVA | SEQ ID NO: 4 |
| | LCDR2 | SASYRFT | SEQ ID NO: 5 |
| | LCDR3 | QHYSTTPFT | SEQ ID NO: 6 |

**Table 9-2 The composition of the heavy and light chains of MTM and MMT**

| Antibod y Number | Immunoglobulin Section | | linker | scFv Section |
|---|---|---|---|---|
| | Heavy chain | Light chain | | |
| MTM | Tezepelumab-HC(SE Q ID NO: 53) | Tezepelumab-LC(SE Q ID NO: 54) | Linker 1 | Mm8Hv(SEQ ID NO: 44)-linker2-Mm8Lv(SEQ ID NO: 45) |
| MMT | m8-HC(SEQ ID NO: 55) | m8-LC(SEQ ID NO: 56) | Linker 1 | MtezeHv(SEQ ID NO: 42)-linker2-MtezeLv(SE Q ID NO: 43) |

The amino acid sequence of the MTM heavy chain (MTM-HC) is:

QMQLVESGGGVVQPGRSLRLSCAASGFTFRTYGMHWVRQAPGKGLEWVAVIWYD
GSNKHYADSVKGRFTITRDNSKNTLNLQMNSLRAEDTAVYYCARAPQWELVHEAFDIW
GQGTMVTVSSASTKGPSVFPLAPCSRSTSESTAALGCLVKDYFPEPVTVSWNSGALTSGV
HTFPAVLQSSGLYSLSSVVTVPSSNFGTQTYTCNVDHKPSNTKVDKTVERKCCVECPPCP
APPVAGPSVFLFPPKPKDTLMISRTPEVTCVVVDVSHEDPEVQFNWYVDGVEVHNAKTK
PREEQFNSTFRVVSVLTVVHQDWLNGKEYKCKVSNKGLPAPIEKTISKTKGQPREPQVY
TLPPSREEMTKNQVSLTCLVKGFYPSDIAVEWESNGQPENNYKTTPPMLDSDGSFFLYSK
LTVDKSRWQQGNVFSCSVMHEALHNHYTQKSLSLSPGKGGGGSGGGGSQVQLVQSGA
EVKKPGASVKVSCKASGYTFTTYYIYWVRQAPGQCLEWMGGSNPSTGGTLDNEKFKT
RVTMTVDKSTSTAYMELSSLRSEDTAVYYCTRSGRYAPFTYWGQGTLVTVSSGGGGSGG
GGSGGGGSGGGGSDIQMTQSPSSLSASVGDRVTITCKASQDVSTSVAWYQQKPGKAPKL
LIYSASYRFTGVPSRFSGSGSGTDFTFTISSLQPEDFATYYCQHYSTTPFTFGCGTKLEIK(S
EQ ID NO: 57)

The amino acid sequence of the MTM light chain (MTM-LC) is:

SYVLTQPPSVSVAPGQTARITCGGNNLGSKSVHWYQQKPGQAPVLVVYDDSDRPS
WIPERFSGSNSGNTATLTISRGEAGDEADYYCQVWDSSSDHVVFGGGTKLTVLGQPKAA

PSVTLFPPSSEELQANKATLVCLISDFYPGAVTVAWKADSSPVKAGVETTTPSKQSNNKY
AASSYLSLTPEQWKSHRSYSCQVTHEGSTVEKTVAPTECS(SEQ ID NO: 58)

The amino acid sequence of the MMT heavy chain (MMT-HC) is:

QVQLVQSGAEVKKPGASVKVSCKASGYTFTTYYIYWVRQAPGQGLEWMGGSNPS
TGGTLDNEKFKTRVTMTVDKSTSTAYMELSSLRSEDTAVYYCTRSGRYAPFTYWGQGTL
VTVSSASTKGPSVFPLAPCSRSTSESTAALGCLVKDYFPEPVTVSWNSGALTSGVHTFPAV
LQSSGLYSLSSVVTVPSSSLGTKTYTCNVDHKPSNTKVDKRVESKYGPPCPPCPAPEFLG
GPSVFLFPPKPKDTLMISRTPEVTCVVVDVSQEDPEVQFNWYVDGVEVHNAKTKPREE
QFNSTYRVVSVLTVLHQDWLNGKEYKCKVSNKGLPSSIEKTISKAKGQPREPQVYTLPP
SQEEMTKNQVSLTCLVKGFYPSDIAVEWESNGQPENNYKTTPPVLDSDGSFFLYSRLTVD
KSRWQEGNVFSCSVMHEALHNHYTQKSLSLSLGKGGGGSGGGGSQMQLVESGGGVVQ
PGRSLRLSCAASGFTFRTYGMHWVRQAPGKCLEWVAVIWYDGSNKHYADSVKGRFTIT
RDNSKNTLNLQMNSLRAEDTAVYYCARAPQWELVHEAFDIWGQGTMVTVSSGGGGSG
GGGSGGGGSGGGGSSYVLTQPPSVSVAPGQTARITCGGNNLGSKSVHWYQQKPGQAPV
LVVYDDSDRPSWIPERFSGSNSGNTATLTISRGEAGDEADYYCQVWDSSSDHVVFGCGT
KLTVL(SEQ ID NO: 59)

The amino acid sequence of the MMT light chain (MMT-LC) is:

DIQMTQSPSSLSASVGDRVTITCKASQDVSTSVAWYQQKPGKAPKLLIYSASYRFTG
VPSRFSGSGSGTDFTFTISSLQPEDFATYYCQHYSTTPFTFGQGTKLEIKRTVAAPSVFIFPP
SDEQLKSGTASVVCLLNNFYPREAKVQWKVDNALQSGNSQESVTEQDSKDSTYSLSSTL
TLSKADYEKHKVYACEVTHQGLSSPVTKSFNRGEC(SEQ ID NO: 60)

### 2. Antibody Expression and Purification

The heavy chain and light chain cDNA sequences of the above bispecific antibody were cloned into the pKS001 vector separately to obtain recombinant expression plasmids for the bispecific antibody. The recombinant plasmids were transiently transfected into ExpiCHO-S cells (Thermo Fisher Scientific), and the bispecific antibodies were purified by Protein A affinity chromatography.

### 3. Antibody Detection

The purity of the antibodies was detected by SDS-PAGE, and the results are shown in Figure 7. Both molecules achieved an electrophoretic purity of above 95%.

### Example 10: Determination of Affinity Constants

### 1. Determination of the affinity of bispecific antibody molecules for hIL-4Rα

The binding affinity (KD) of the 2 antibody molecules for hIL-4Rα was determined at 25°C using real-time surface plasmon resonance analysis (Biacore 8K). Firstly, an anti-mouse Fc IgG antibody was coupled onto a CM5 chip to create an antibody capture surface. The antibody molecules were then coupled onto this anti-mouse Fc IgG antibody at a flow rate of 10 µL/min for 30 seconds. The analyte (hIL-4Rα.ECD) was prepared into 6 serial 1:2 dilutions and sequentially analyzed, with an association time of 180 seconds, a dissociation time of 1800 seconds, and a flow rate of 30 µL/min. Finally, kinetic analysis was performed using BIA evaluation software to determine the equilibrium association constant (Ka), equilibrium dissociation constant (Kd), and affinity constant (KD). The measurement results are shown in Table 10. The 2 constructed bispecific antibody molecules achieved affinity for hIL-4Rα comparable to that of the control molecules m8 and Dupilumab molecules, indicating their high specificity.

**Table 10 Affinity data of antibodies for hIL-4Rα**

| Antibodies | Antigen | Ka (1/Ms) | Kd (1/s) | KD (M) |
|---|---|---|---|---|
| MTM | hIL4Rα | 5.28E+05 | 3.48E-05 | 6.59E-11 |
| MMT | | 7.27E+05 | 4.35E-05 | 5.98E-11 |
| m8 | | 2.85E+05 | 1.39E-05 | 4.89E-11 |
| Dupilumab | | 9.24E+05 | 5.70E-05 | 6.16E-11 |

### 2. Determination of the affinity of bispecific antibody molecules for hTSLP

The binding affinity (KD) of the 2 antibody molecules for hTSLP was determined at 25°C using real-time surface plasmon resonance analysis (Biacore 8K). Firstly, an anti-mouse Fc IgG antibody was coupled onto a CM5 chip to form an antibody capture surface. The antibody molecules were then coupled onto this anti-mouse Fc IgG antibody at a flow rate of 10 µL/min for 30 seconds. The analyte (hTSLP) was prepared into 6 serial 1:2 dilutions and sequentially analyzed, with an association time of 180 seconds, a dissociation time of 1800 seconds, and a flow rate of 30 µL/min. Finally, kinetic analysis was performed using BIA evaluation software to determine the equilibrium association constant (Ka), equilibrium dissociation constant (Kd), and affinity constant (KD). The obtained results are shown in Table 11. The 2 bispecific antibody molecules maintained an affinity level comparable to that of the control molecule Tezepelumab.

**Table 11 Affinity data of antibodies for hTSLP**

| Antibodies | Antigen | Ka (1/Ms) | Kd (1/s) | KD (M) |
|---|---|---|---|---|
| MTM | hTSLP | 1.18E+07 | 3.50E-05 | 2.96E-12 |
| MMT | | 3.14E+06 | 5.88E-05 | 1.88E-11 |
| Tezepelumab | | 1.11E+07 | 2.16E-05 | 1.95E-12 |

### 3. Simultaneous binding of MTM and MMT antibodies to hIL-4Rα and hTSLP antigens

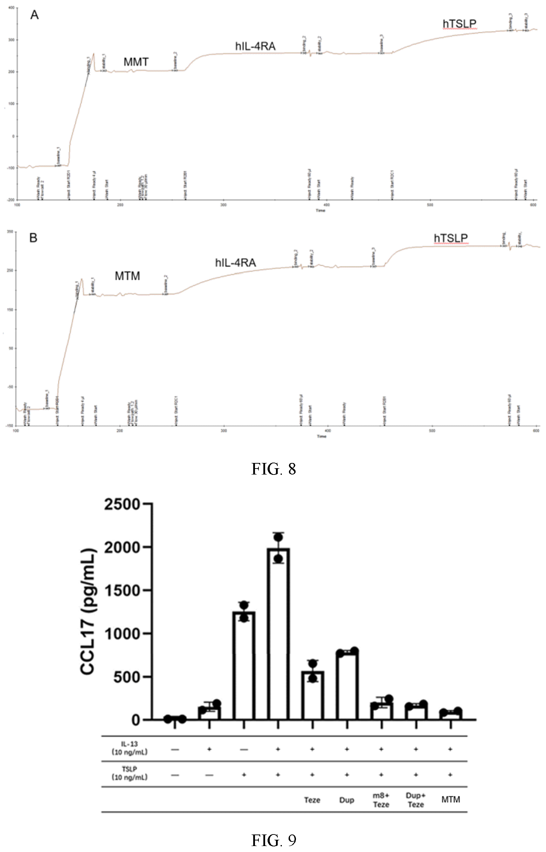

The ability of MTM and MMT molecules to simultaneously bind hIL-4Rα and hTSLP antigens was investigated at 25°C using real-time surface plasmon resonance analysis (Biacore 8K). Firstly, an anti-mouse Fc IgG antibody was coupled onto a CM5 chip to result in an antibody capture surface. Subsequently, the MTM and MMT antibody molecules were respectively coupled onto the anti-mouse Fc IgG antibody at a flow rate of 10 µL/min for 30 seconds. The analytes, hIL-4Rα and hTSLP, were then sequentially flowed over the chip to test their binding capability, with an association time of 180 seconds. The binding sensorgrams confirmed that both antibody molecules retained the ability to bind hTSLP after binding to the hIL-4Rα antigen. The results are shown in Figure 8. Figure 8A demonstrates that after coupling the MMT molecule and sequentially injecting hIL-4Rα and hTSLP molecules, MMT was able to bind both. Figure 8B demonstrates that after coupling the MTM molecule and sequentially injecting hIL-4Rα and hTSLP molecules, MTM was also able to bind both. This indicates that the MTM and MMT antibody molecules can simultaneously bind to hIL-4Rα and hTSLP antigens.

### Example 11: Evaluation of IL-4 and IL-13 Cell Activity

### 1. Evaluation of the inhibitory activity of bispecific antibody molecules on IL-4 using a reporter gene method (HEK-293T.STAT6-GFP cells)

HEK-293T.STAT6-GFP cells were resuspended in TransDetect BrightFluore DMEM + 10% FBS medium, and the cell density was adjusted to 5E5 cells/mL. The cells were seeded into a black 96-well plate at 100 µL/well and incubated at 37°C for 24 hours. hIL-4 was diluted to a concentration of 1.0 ng/mL using TransDetect BrightFluore DMEM + 10% FBS medium. Dilute the antibody using the pre-diluted hIL-4 solution, with 11 serial 1:4 dilutions. Then, add 100 µL/well to a 96-well plate, mixed gently, and incubated at 37°C for another 24 hours. Fluorescence signal intensity was measured using a microplate reader with an excitation wavelength of 488 nm and an emission wavelength of 520 nm. The results are shown in Table 12, indicating that the MTM molecule exhibited an inhibitory activity against hIL-4 comparable to that of the positive control molecule m8.

**Table 12 hIL-4 inhibitory activity data from the reporter gene assay**

| Antibodies | IC50(pM) |
|---|---|
| MTM | 170 |
| m8 | 210 |

### 2.Evaluation of the inhibitory activity of bispecific antibody molecules on IL-13 using a reporter gene method (HEK-293T.STAT6-GFP cells)

HEK-293T.STAT6-GFP cells were prepared, resuspended in TransDetect BrightFluore DMEM + 10% FBS medium, and the cell density was adjusted to 5E5 cells/mL. The cells were seeded into a black 96-well plate at 100 µL/well and incubated at 37°C for 24 hours. hIL-13 was diluted to a concentration of 30 ng/mL using TransDetect BrightFluore DMEM + 10% FBS medium. Dilute the antibody using the pre-diluted hIL-13 solution, with 11 serial 1:4 dilutions. Then, add 100 µL/well to a 96-well plate, mixed gently, and incubated at 37°C for another 24 hours. Fluorescence signal intensity was measured using a microplate reader with an excitation wavelength of 488 nm and an emission wavelength of 520 nm. The results are shown in Table 13, indicating that the MMT and MTM molecules exhibited inhibitory activity against hIL-13 comparable to those of the positive control molecules m8 and Dupilumab.

**Table 13 hIL-13 inhibitory activity data from the reporter gene assay**

| Antibodies | IC50 (pM) |
|---|---|
| MTM | 66.5 |
| MMT | 133 |
| m8 | 80 |
| Dupilumab | 41 |

### Example 12: Evaluation of the inhibitory activity of bispecific antibody molecules on TSLP using reporter gene assay (HEK-293T-hTSLPR-IL7Ra-STAT5-Luciferase cells)

HEK-293T-hTSLPR-IL7Ra-STAT5-Luciferase cells were resuspended in DMEM + 10% FBS medium, and the cell density was adjusted to 5E5 cells/mL. The cells were seeded into a black 96-well plate at 100 µL/well and incubated at 37°C for 24 hours. The hTSLP was diluted to a concentration of 5 ng/mL using DMEM + 10% FBS medium. Dilute the antibody using the pre-diluted hTSLP solution, with 11 serial 1:3 dilutions. Then, add 100 µL/well to a 96-well plate, mixed gently, and incubated at 37°C for another 24 hours. The luminescent substrate was removed out from 4°C and returned to room temperature in advance. After the incubation, the 96-well plate was placed at room temperature for 10 minutes. Subsequently, 100 µL of luminescent substrate was added to each well and incubated for 2 minutes at room temperature protected from light. The luminescence signal intensity was measured using a microplate reader. The results are shown in Table 14, indicating that the MTM and MMT molecules exhibited inhibitory activities against hTSLP comparable to that of the positive control molecule Tezepelumab.

**Table 14 hTSLP inhibitory activity data from the reporter gene assay**

| Antibodies | IC50 (nM) |
|---|---|
| MTM | 0.14 |
| MMT | 0.30 |
| Tezepelumab | 0.12 |

### Example 13: Inhibitory activity of the MTM antibody on chemokine 17 (CCL17) expression in human PBMCs

Human PBMCs were purchased from Shanghai Aoneng Biotechnology Co., Ltd. The human PBMCs were thawed with RPMI 1640 + 10% FBS medium and incubated at 37°C for 2 hours. After cell activation, the cells were washed twice with RPMI 1640 medium without FBS, then resuspended in RPMI 1640 medium without FBS for counting. Cells were seeded into a 96-well plate at 100,000 cells per well in a volume of 100 µL. IL-13 and TSLP were each diluted to a concentration of 20 ng/mL using RPMI 1640 medium without FBS. The antibody molecules were diluted to equimolar concentrations using the diluent solution. The prepared drug solutions were then added to the corresponding wells. The plate was incubated at 37°C for another 48 hours. Cell culture supernatants were collected, and the concentration of CCL17 in the supernatants was detected using a sandwich ELISA. The results are shown in Figure 9. PBMCs stimulated with either IL-13 or TSLP alone secreted CCL17 in varying degree, the stimulatory effect was the strongest when IL-13 and TSLP were added simultaneously. Under co-stimulation with IL-13 and TSLP, Tezepelumab (Teze) and Dupilumab (Dup) could inhibit CCL17 secretion partially if added separately, compared to the control group without antibody addition. Teze/Dup combination, or Teze/m8 combination could significantly inhibit CCL17 secretion when added silultaneously. The MTM bispecific antibody was added alone and could achieve an inhibitory effect comparable to or even superior to that of the monoclonal antibodies combination treatments.

### Example 14: In vivo efficacy evaluation of the MTM antibody in a B-hIL4/IL4R humanized mouse asthma model

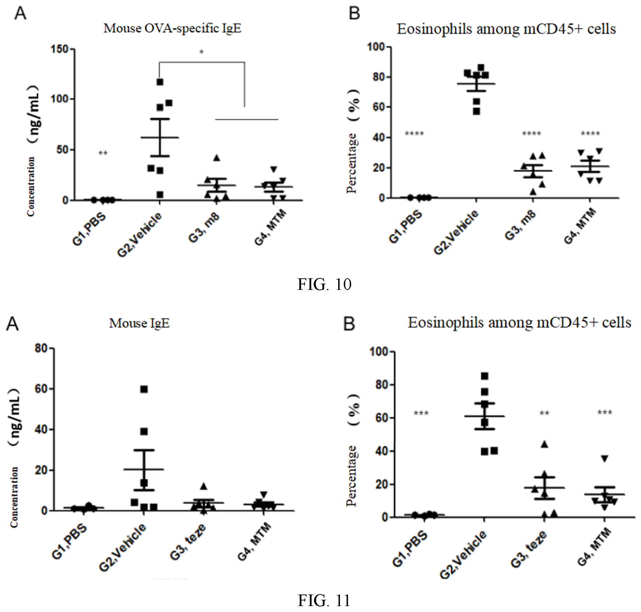

OVA (Ovalbumin, Sigma, Cat# A5503) was used as an inducer to establish an asthma model in B-hIL4/IL4R humanized mice (doi.org/10.1016/j.imbio.2020.151998). The experimental mice were divided into 4 groups: G1 (non-modeling group) with 4 animals, and G2-G4 with 6 animals respectively. G1 served as the blank control group. G2 was the isotype control group. G3 was the m8 control antibody treatment group, with a dosage of 25 mg/kg. G4 was the MTM test sample treatment group, with a dosage of 34 mg/kg. The administration route was intraperitoneal injection. Administration was conducted from the 13^{th} day of the modeling procedure, followed by the 16^{th} day, the 20^{th} day, and the 23^{th} day, a total of 4 times. On the 26^{th} day, blood collection and dissection were performed on the animals. The eosinophil and IgE levels were analyzed. The results are shown in Figure 10, indicating that the MTM molecule exhibited comparable pharmacodynamic activity to that of the m8 control molecule in vivo asthma model.

### Example 15: In vivo efficacy evaluation of the MTM antibody in a B-hTSLP/TSLPR/hIL7R humanized mouse asthma model

TSLP (ACRO, Cat# TSP-H52Hb) combined with OVA (Ovalbumin, Sigma, Cat# A5503) was used as an inducer to establish an asthma model in B-hTSLP/TSLPR/hIL7R humanized mice. On days 0, 2, 4, 6, 8, 10, and 12 separately, animals in group G1 were administered 20 µL PBS via intranasal administration, each animal in groups G2-G4 was administered 20 µL mixture solution of a TSLP+OVA (1µg + 10µg) via intranasal administration, for a total of 7 intranasal inductions. Group G1 was served as the blank control group. Group G2 was the isotype control group. Group G3 was the Tezepelumab (10 mg/kg) control antibody treatment group. Group G4 was the test article treatment group of TM (14 mg/kg). Administration was conducted intraperitoneally on day 1 and 6, a total of twice administrations. After the administration period, blood samples and dissection were performed on the animals. The eosinophil and IgE levels were analyzed. The experimental results are shown in Figure 11 indicating that the MTM molecule exhibited comparable pharmacodynamic activity to that of the Tezepelumab control molecule in vivo asthma model.

### Example 16: Preparation of high-concentration MTM samples

### 1. Construction of cells stably expressing the MTM molecule

The heavy chain and light chain cDNA sequences of the MTM molecule were separately cloned into the pKS001 vector to obtain a recombinant expression plasmid for bispecific antibody expression. Using MSX pressure selection, the recombinant plasmid was transfected into CHO-K1 cells (Merck). Stable transfectant cell pool expressing MTM was obtained through pressure selection. The stable transfectant cell pool was subjected to fermentation culture, and the fermentation supernatant expressing MTM was harvested.

### 2. Ultrafiltration concentration of the MTM molecule

After purification of the fermentation supernatant by Protein A affinity chromatography, a sample with purity greater than 95% was collected. The sample was then concentrated by ultrafiltration using a 30 kDa centrifugal filter unit and buffer-exchanged into 20 mM sodium acetate, pH 5.0, containing 100 mM L-proline buffer solution. The final concentration reached more than 100 mg/mL.

### Example 17: Stability evaluation experiment

The concentrated samples were diluted to a concentration of 100 mg/mL and 10 mg/mL using 20 mM sodium acetate, pH 5.0, containing 100 mM L-proline buffer solution. Stability studies were conducted at 4°C, 25°C, and 40°C. The studies at 4°C and 25°C were performed using the 100 mg/mL concentration, while the study at 40°C was performed using the 10 mg/mL concentration. Sample purity was determined by SEC-HPLC. The results are shown in Table 15. After 4 weeks of accelerated testing at high temperature, the change of monomer purity was less than 5%. Under the 4°C and 25°C conditions, the monomer content decreased by less than 2%. These results indicate that the MTM bispecific antibody molecule possesses good stability.

**Table 15 Stability evaluation results**

| Temperature(°C) | Concentration(mg/mL) | Time(Weeks) | Monomer(%) | Δ Monomer(%) |
|---|---|---|---|---|
| T0 | 100 | 0 | 96.4 | 0.0 |
| 4.0 | 100 | 4 | 95.9 | -0.5 |
| 25.0 | 100 | 4 | 94.9 | -1.5 |
| 40.0 | 10 | 2 | 94.7 | -1.7 |
| | 10 | 4 | 93.7 | -2.7 |

It should be understood that the above embodiments are merely illustrative examples adopted to explain the principles of the present invention, and the present invention is not limited thereto. For those skilled in the art, various modifications and improvements can be made without departing from the principle and essence of the present invention, and these modifications and improvements are also deemed to fall within the protection scope of the present invention.

## Claims

1. An anti-IL-4Rα antibody or antigen-binding fragment thereof, **characterized in that**, it comprises a heavy chain complementarity determining region and a light chain complementarity determining region,
the heavy chain complementarity determining region comprises HCDR1, HCDR2, and HCDR3, wherein the HCDR1 is the amino acid sequence shown in SEQ ID NO: 1 or SEQ ID NO: 12; the HCDR2 is the amino acid sequence shown in SEQ ID NO: 2, SEQ ID NO: 7, SEQ ID NO: 10, or SEQ ID NO: 13; the HCDR3 is the amino acid sequence shown in SEQ ID NO: 3, SEQ ID NO: 9, or SEQ ID NO: 14;
the light chain complementarity determining region comprises LCDR1, LCDR2, and LCDR3, wherein the LCDR1 is the amino acid sequence shown in SEQ ID NO: 4 or SEQ ID NO: 15; the LCDR2 is the amino acid sequence shown in SEQ ID NO: 5 or SEQ ID NO: 16; the LCDR3 is the amino acid sequence shown in SEQ ID NO: 6, SEQ ID NO: 8, SEQ ID NO: 11, or SEQ ID NO: 17.

2. The anti-IL-4Rα antibody or antigen-binding fragment thereof according to claim 1, **characterized in that**, it comprises:
(1) a heavy chain complementarity determining region comprising HCDR1 as shown in SEQ ID NO: 1, HCDR2 as shown in SEQ ID NO: 2, and HCDR3 as shown in SEQ ID NO: 3; and
a light chain complementarity determining region comprising LCDR1 as shown in SEQ ID NO: 4, LCDR2 as shown in SEQ ID NO: 5, and LCDR3 as shown in SEQ ID NO: 6; or
(2) a heavy chain complementarity determining region comprising HCDR1 as shown in SEQ ID NO: 1, HCDR2 as shown in SEQ ID NO: 7, and HCDR3 as shown in SEQ ID NO: 3; and
a light chain complementarity determining region comprising LCDR1 as shown in SEQ ID NO: 4, LCDR2 as shown in SEQ ID NO: 5, and LCDR3 as shown in SEQ ID NO: 8; or
(3) a heavy chain complementarity determining region comprising HCDR1 as shown in SEQ ID NO: 1, HCDR2 as shown in SEQ ID NO: 7, and HCDR3 as shown in SEQ ID NO: 9; and
a light chain complementarity determining region comprising LCDR1 as shown in SEQ ID NO: 4, LCDR2 as shown in SEQ ID NO: 5, and LCDR3 as shown in SEQ ID NO: 8; or
(4) a heavy chain complementarity determining region comprising HCDR1 as shown in SEQ ID NO: 1, HCDR2 as shown in SEQ ID NO: 10, and HCDR3 as shown in SEQ ID NO: 3; and
a light chain complementarity determining region comprising LCDR1 as shown in SEQ ID NO: 4, LCDR2 as shown in SEQ ID NO: 5, and LCDR3 as shown in SEQ ID NO: 11; or
(5) a heavy chain complementarity determining region comprising HCDR1 as shown in SEQ ID NO: 12, HCDR2 as shown in SEQ ID NO: 13, and HCDR3 as shown in SEQ ID NO: 14; and
a light chain complementarity determining region comprising LCDR1 as shown in SEQ ID NO: 15, LCDR2 as shown in SEQ ID NO: 16, and LCDR3 as shown in SEQ ID NO: 17.

3. The anti-IL-4Rα antibody or antigen-binding fragment thereof according to claim 1, **characterized in that**, it further comprises:
framework region sequences derived from human germline heavy chain IGHV1-46*01 + IGHJ1*01, or back-mutated sequences having at least 90% identity thereof; or
framework region sequences derived from human germline light chain IGKV1-39*01 + IGKJ2*01, or back-mutated sequences having at least 90% identity thereof.

4. The anti-IL-4Rα antibody or antigen-binding fragment thereof according to claim 1, **characterized in that**, the antibody or antigen-binding fragment thereof is a murine antibody, a chimeric antibody, a human antibody, a humanized antibody, a bispecific antibody, or an antigen-binding fragment thereof.

5. The anti-IL-4Rα antibody or antigen-binding fragment thereof according to claim 1, **characterized in that**, it comprises:
(1) a variable region of heavy chain comprising the amino acid sequence as shown in SEQ ID NO: 28 and a variable region of light chain comprising the amino acid sequence as shown in SEQ ID NO: 29;
(2) a variable region of heavy chain comprising the amino acid sequence as shown in SEQ ID NO: 30 and a variable region of light chain comprising the amino acid sequence as shown in SEQ ID NO: 31;
(3) a variable region of heavy chain comprising the amino acid sequence as shown in SEQ ID NO: 32 and a variable region of light chain comprising the amino acid sequence as shown in SEQ ID NO: 33;
(4) a variable region of heavy chain comprising the amino acid sequence as shown in SEQ ID NO: 18 and a variable region of light chain comprising the amino acid sequence as shown in SEQ ID NO: 19; or
(5) a variable region of heavy chain comprising the amino acid sequence as shown in SEQ ID NO: 20 and a variable region of light chain comprising the amino acid sequence as shown in SEQ ID NO: 21.

6. The anti-IL-4Rα antibody or antigen-binding fragment thereof according to claim 5, **characterized in that**, it comprises an amino acid sequence having 90% or more identity.

7. A fusion protein, comprising the anti-IL-4Rα antibody or antigen-binding fragment thereof according to any one of claims 1-6.

8. A polynucleotide encoding the antibody or antigen-binding fragment thereof according to any one of claims 1-6.

9. An expression vector comprising the polynucleotide according to claim 8.

10. A host cell comprising the vector according to claim 8, wherein the host cell is a prokaryotic cell or a eukaryotic cell.

11. A pharmaceutical composition comprising the anti-IL-4Rα antibody or antigen-binding fragment thereof according to any one of claims 1-6 or the fusion protein according to claim 7, and pharmaceutically acceptable excipients.

12. Use of the anti-IL-4Rα antibody or antigen-binding fragment thereof according to any one of claims 1-6, the fusion protein according to claim 7, or the pharmaceutical composition according to claim 11 in the preparation of a medicament for treating an allergic disease.

13. The use according to claim 12, wherein the allergic disease is atopic dermatitis, asthma, allergic rhinitis, certain cases of chronic nasosinusitis with nasal polyps, or chronic obstructive pneumonia.

14. A bispecific antibody against IL-4Rα and TSLP, **characterized in that**, the bispecific antibody comprises the anti-IL-4Rα antibody or antigen-binding fragment thereof selected from any one of claims 1-6.

15. The bispecific antibody according to claim 14, **characterized in that**, the bispecific antibody comprises an immunoglobulin IgG and a single-chain antibody scFv, wherein the bispecific antibody is selected from one of the following groups:
(1) the immunoglobulin IgG comprises an anti-TSLP antibody or antigen-binding fragment thereof, and the single-chain antibody scFv comprises the anti-IL-4Rα antibody or antigen-binding fragment thereof selected from any one of claims 1-6; or
(2) the immunoglobulin IgG comprises the anti-IL-4Rα antibody or antigen-binding fragment thereof selected from any one of claims 1-6, and the single-chain antibody scFv comprises an anti-TSLP antibody or antigen-binding fragment thereof.

16. The bispecific antibody according to claim 15, **characterized in that**, the anti-TSLP antibody or antigen-binding fragment thereof comprises a variable region of heavy chain and a variable region of light chain, wherein
the variable region of heavy chain comprises HCDR1, HCDR2, and HCDR3, wherein the HCDR1 is the amino acid sequence shown in SEQ ID NO: 48; the HCDR2 is the amino acid sequence shown in SEQ ID NO: 49; the HCDR3 is the amino acid sequence shown in SEQ ID NO: 50;
the variable region of light chain comprises LCDR1, LCDR2, and LCDR3, wherein the LCDR1 is the amino acid sequence shown in SEQ ID NO: 51; the amino acid sequence of LCDR2 is DDS; the LCDR3 is the amino acid sequence shown in SEQ ID NO: 52.

17. The bispecific antibody according to claim 15, **characterized in that** the anti-IL-4Rα antibody or antigen-binding fragment thereof comprises a variable region of heavy chain and a variable region of light chain, wherein,
the variable region of heavy chain comprises HCDR1, HCDR2, and HCDR3, wherein the HCDR1 is the amino acid sequence shown in SEQ ID NO: 1; the HCDR2 is the amino acid sequence shown in SEQ ID NO: 2; the HCDR3 is the amino acid sequence shown in SEQ ID NO: 3;
the variable region of light chain comprises LCDR1, LCDR2, and LCDR3, wherein the LCDR1 is the amino acid sequence shown in SEQ ID NO: 4; the LCDR2 is the amino acid sequence shown in SEQ ID NO: 5; the LCDR3 is the amino acid sequence shown in SEQ ID NO: 6.

18. The bispecific antibody according to claim 15, **characterized in that**, the anti-TSLP antibody or antigen-binding fragment thereof comprises a variable region of heavy chain and a variable region of light chain, wherein,
the variable region of heavy chain has the amino acid sequence shown in SEQ ID NO: 42, and the variable region of light chain has the amino acid sequence shown in SEQ ID NO: 43; or,
the variable region of heavy chain has the amino acid sequence shown in SEQ ID NO: 34, and the variable region of light chain has the amino acid sequence shown in SEQ ID NO: 36.

19. The bispecific antibody according to claim 15, **characterized in that**, the anti-IL-4Rα antibody or antigen-binding fragment thereof comprises a variable region of heavy chain and a variable region of light chain, wherein,
the variable region of heavy chain has the amino acid sequence shown in SEQ ID NO: 44, and the variable region of light chain has the amino acid sequence shown in SEQ ID NO: 45; or
the variable region of heavy chain has the amino acid sequence shown in SEQ ID NO: 28, and the variable region of light chain has the amino acid sequence shown in SEQ ID NO: 29.

20. The bispecific antibody according to claim 15, **characterized in that**, the immunoglobulin IgG and the single-chain antibody scFv are connected via a linker, wherein the linker is (GGGGS)n, with n being a positive integer from 1 to 5, preferably the amino acid sequence as shown in SEQ ID NO: 46.

21. The bispecific antibody according to claim 15, **characterized in that**, the single-chain antibody scFv further comprises a linker connecting the variable region of heavy chain and the variable region of light chain of the scFv, wherein the linker is (GGGGS)n, with n being a positive integer from 1 to 5, preferably the amino acid sequence as shown in SEQ ID NO: 47.

22. The bispecific antibody according to claim 15, **characterized in that** the bispecific antibody is selected from any one of the following groups:
(1) the immunoglobulin IgG comprises a variable region of heavy chain as shown in SEQ ID NO: 34, a constant region of heavy chain as shown in SEQ ID NO: 35, a variable region of light chain as shown in SEQ ID NO: 36, and a constant region of light chain as shown in SEQ ID NO: 37, and the single-chain antibody scFv portion has a variable region of heavy chain as shown in SEQ ID NO: 44 and a variable region of light chain as shown in SEQ ID NO: 45; or
(2) the immunoglobulin IgG comprises a variable region of heavy chain as shown in SEQ ID NO: 28, a constant region of heavy chain as shown in SEQ ID NO: 40, a variable region of light chain as shown in SEQ ID NO: 29, and a constant region of light chain as shown in SEQ ID NO: 41, and the single-chain antibodyscFv portion has a variable region of heavy chain as shown in SEQ ID NO: 42 and a variable region of light chain as shown in SEQ ID NO: 43.

23. The bispecific antibody according to claim 22, **characterized in that**, the bispecific antibody is selected from any one of the following groups:
(1) the bispecific antibody comprises a heavy chain as shown in SEQ ID NO: 57 and a light chain as shown in SEQ ID NO: 58; or
(2) the bispecific antibody comprises a heavy chain as shown in SEQ ID NO: 59 and a light chain as shown in SEQ ID NO: 60.

24. A pharmaceutical composition comprising the bispecific antibody or antigen-binding fragment thereof according to any one of claims 14-23 and pharmaceutically acceptable excipients.

25. Use of the bispecific antibody according to any one of claims 14-23 or the pharmaceutical composition according to claim 24 in the preparation of a medicament for the treatment of moderate-to-severe asthma, moderate-to-severe atopic dermatitis, allergic rhinitis, certain cases of chronic nasosinusitis with nasal polyps, or chronic obstructive pulmonary diseases.
